(19) Europäisches Patentamt
European Patent Office
Office européen des brevets

(11) EP 1 576 945 B1

(12) # EUROPÄISCHE PATENTSCHRIFT

(45) Veröffentlichungstag und Bekanntmachung des Hinweises auf die Patenterteilung:
**19.12.2012 Patentblatt 2012/51**

(51) Int Cl.:
***A61K 8/898*** *(2006.01)* ***A61Q 5/00*** *(2006.01)*
***A61Q 5/02*** *(2006.01)*

(21) Anmeldenummer: **05005717.3**

(22) Anmeldetag: **16.03.2005**

(54) **Kosmetische oder pharmazeutische Mittel, enthaltend modifizierte Polyorganosiloxane**

Cosmetic or pharmaceutic composition comprising modified polyorganosiloxane

Composition cosmétique ou pharmaceutique comprenant un polyorganosiloxane modifié

(84) Benannte Vertragsstaaten:
**DE FR GB IT**

(30) Priorität: **20.03.2004 DE 102004013795**

(43) Veröffentlichungstag der Anmeldung:
**21.09.2005 Patentblatt 2005/38**

(73) Patentinhaber: **Clariant Produkte (Deutschland) GmbH**
**65929 Frankfurt am Main (DE)**

(72) Erfinder:
- **Meder, Markus, Dr.**
  **97956 Werbach (DE)**
- **Klug, Peter, Dr.**
  **63762 Grossostheim (DE)**
- **Henning, Torsten, Dr.**
  **19057 Schwerin (DE)**
- **Simsch, Waltraud**
  **65779 Kelkheim (DE)**
- **Haala, Sabine**
  **63457 Hanau (DE)**
- **Müller, Carsten**
  **60486 Frankfurt am Main (DE)**

(56) Entgegenhaltungen:
**EP-A- 1 512 391** **WO-A-02/092666**
**WO-A1-03/066007**

- **PATENT ABSTRACTS OF JAPAN Bd. 1999, Nr. 04, 30. April 1999 (1999-04-30) & JP 11 012152 A (TOSHIBA SILICONE CO LTD), 19. Januar 1999 (1999-01-19)**
- **PATENT ABSTRACTS OF JAPAN Bd. 1997, Nr. 11, 28. November 1997 (1997-11-28) & JP 09 194335 A (SHIN ETSU CHEM CO LTD), 29. Juli 1997 (1997-07-29)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 576 945 B1

## Beschreibung

Gegenstand der Erfindung ist die Verwendung kosmetischer oder pharmazeutischer Mittel, enthaltend Aminopolyorganosiloxane, die mit Alkylpolyglycolethergruppen substituiert sind, zum Schutz und zur Erhaltung der Farbe in gefärbten Keratinfasern.

Es ist bekannt, dass Aminosiloxane mit primären und sekundären Stickstoffgruppen und teilweise vorhandenen reaktiven Silanolgruppen als Konditioniermittel in Haarshampoo-Formulierungen eingearbeitet werden. Diese Produkte sind nicht wasserlöslich und können nur in Gegenwart von grenzflächenaktiven Substanzen eingearbeitet werden. Um die Wasserlöslichkeit zu verbessern, können die Aminosiloxane zusätzlich mit Polyoxyalkylengruppen substituiert werden, wie in US 5,075,403 beschrieben. Nachteilig ist, dass diese hochviskos und nur in Verdünnung handhabbar sind, bei der Anwendung größtenteils in der wässrigen Phase verbleiben und nicht in gewünschter Weise auf das Haar aufziehen. In WO 02/092666 werden Aminopolyorganosiloxane und deren Verwendung zur weichmachenden Ausrüstung von Textilfasermaterialien beansprucht.

Die Aufgabe der vorliegenden Erfindung bestand darin, Mittel für kosmetische oder pharmazeutische Erzeugnisse herzustellen, die wasserlöslich, emulgierbar, kompatibel mit in kosmetischen Mitteln gängigen Zusatz- und Hilfsstoffen sind, leicht in Formulierungen eingearbeitet werden können, ein möglichst klares Erscheinungsbild ergeben und eine weichmachende Wirkung zeigen. Darüber hinaus sollen die Mittel eine gute Substantivität aufweisen und für getönte oder gefärbte Haare eine Verbesserung des Farbaufziehverhaltens und eine Erhöhung der Farbstabilität und Formbeständigkeit bewirken.

Überraschenderweise wurde gefunden, dass substituierte Aminopolyorganosiloxane ($S_H$) mit substituierten Aminogruppen, die über Alkylenbrücken oder Mono- oder Oligo-(alkylenamino)-alkylenbrücken an Siliciumatome des Polysiloxangrundgerüstes gebunden sind, wobei
die in den Aminopolyorganosiloxanen ($S_H$) vorkommenden Aminogruppen mindestens teilweise mit einem Rest der Formel (ε)

$$T\text{- }CH_2\text{- }CHOH\text{ - }CH_2\text{-} \qquad (\varepsilon),$$

worin T den Rest eines tensiden Monoalkoholpolyglykoläthers mit Emulgatorcharakter bedeutet und die Alkoholpolyglykoläther T-H solche der folgenden durchschnittlichen Formel

$$R_1 \left( O - X \right)_q OH \qquad \textbf{(II)}$$

sind,
worin

$R_1$ einen alkylaromatischen oder aliphatischen Kohlenwasserstoffrest mit 9 bis 24 Kohlenstoffatomen,
X $C_{2-4}$-Alkylen

und

q 4 bis 50,

bedeuten, wobei mindestens 80 % der q Alkylenoxygruppen Äthylenoxyeinheiten sind, substituiert sind,
die in den Aminopolyorganosiloxanen ($S_H$) vorkommenden Aminogruppen im durchschnittlichen Verhältnis von mindestens 1,5 Resten der Formel (ε) pro Sigebundene Aminoalkylgruppe oder Amino-mono- oder -oligo-(alkylenamino)-alkylGruppe substituiert sind, und
vorhandene Aminogruppen gegebenenfalls mindestens teilweise zu Amidgruppen acyliert und/oder alkyliert und/oder benzyliert und/oder protoniert sind,
eine hervorragende Substantivität, sowie gute konditionierende und farberhaltende bis farbvertiefende Effekte, insbesondere gegenüber Haaren, zeigen.

Gegenstand der vorliegenden Erfindung ist daher die Verwendung kosmetischer oder pharmazeutischer Mittel enthaltend ein oder mehrere substituierte Aminopolyorganosiloxane ($S_H$) mit substituierten Aminogruppen, die über Alkylenbrücken oder Mono- oder Oligo-(alkylenamino)-alkylenbrücken an Siliciumatome des Polysiloxangrundgerüstes gebunden sind, wobei die in den Aminopolyorganosiloxanen ($S_H$) vorkommenden Aminogruppen mindestens teilweise mit einem Rest der Formel (ε)

$$T\text{-}CH_2\text{-}CHOH\text{-}CH_2\text{-} \qquad (\varepsilon),$$

worin T den Rest eines tensiden Monoalkoholpolyglykoläthers mit Emulgatorcharakter bedeutet und die Alkoholpolyglykoläther T-H solche der folgenden durchschnittlichen Formel

$$R_1 \left( O-X \right)_q OH \qquad (II)$$

sind,
worin

$R_1$ einen alkylaromatischen oder aliphatischen Kohlenwasserstoffrest mit 9 bis 24 Kohlenstoffatomen,
X $C_{2-4}$-Alkylen

und

q 4 bis 50,

bedeuten, wobei mindestens 80 % der q Alkylenoxygruppen Äthylenoxyeinheiten sind, substituiert sind,
die in den Aminopolyorganosiloxanen ($S_H$) vorkommenden Aminogruppen im durchschnittlichen Verhältnis von mindestens 1,5 Resten der Formel (ε) pro Sigebundene Aminoalkylgruppe oder Amino-mono- oder -oligo-(alkylenamino)-alkylGruppe substituiert sind, und
vorhandene Aminogruppen gegebenenfalls mindestens teilweise zu Amidgruppen acyliert und/oder alkyliert und/oder benzyliert und/oder protoniert sind,
zum Schutz und zur Erhaltung der Farbe in gefärbten Keratinfasern.

Das Farbaufziehverhalten von Haarfärbemitteln kann durch Aminopolyorganosiloxane ($S_H$) verbessert werden. In Haarstylingmitteln ist zudem eine volumen- und glanzgebende Wirkung der Aminopolyorganosiloxane ($S_H$) signifikant. Vorteilhaft ist ausserdem die gute Wasserlöslichkeit, aber auch die gute Kompatibilität mit hydrophoben Komponenten, ein gutes Lösungs-, Dispergier- und Emulgiervermögen, ein günstiges Viskositätsverhalten mit niedriger Viskosität und guter Einarbeitbarkeit in hochkonzentrierter Form, sowie ein klares Erscheinungsbild der efindungsgemäß eingesetzten Aminopolyorganosiloxane ($S_H$). Aminopolyorganopolysiloxane ($S_H$) zeichnen sich durch eine gute Hautsensorik aus und zeigen ein gutes Spreitungsvermögen, sowie eine hervorragende Gleit- und Carrierwirkung. Darüberhinaus sind sie unempfindlich gegenüber Hitze, UV- und IR-Strahlung. Sie sind somit wertvolle Bestandteile für Haarpflege- und Haarreinigungsmittel und Haarfärbemittel.

Bei den Mitteln kann es sich beispielsweise um wässrige, wässrig-alkoholische, wässrig-tensidische oder alkoholische Mittel oder um Mittel auf Ölbasis, inclusive Mittel auf Ölbasis in wasserfreier Form oder um Emulsionen, Suspensionen oder Dispersionen handeln.

In einer bevorzugten Ausführungsform der Erfindung liegen die kosmetischen oder pharmazeutischen Mittel in wässriger, wässrig-alkoholischer, alkoholischer oder wässrig-tensidischer Form vor oder stellen Mittel auf Basis von Öl, insbesondere wasserfreie Mittel auf Basis von Öl, dar oder liegen als Emulsion, Suspension oder Dispersion vor und zwar in Form von Fluids, Schäumen, Sprays, Gelen, Mousse, Lotionen, Cremes oder Pudern.

Unter Verwendung der Aminopolyorganosiloxane ($S_H$) lassen sich klare, viskose, wässrige, wässrig-alkoholische, wässrig-tensidische Mittel, alkoholische Mittel oder auch Mittel auf Ölbasis mit einem sehr ästhetischen Erscheinungsbild herstellen.

In einer weiteren bevorzugten Ausführungsform der Erfindung handelt es sich bei den kosmetischen oder pharmazeutischen Mitteln um kosmetische oder pharmazeutische Formulierungen.
Die in den kosmetischen und pharmazeutischen Mitteln eingesetzten substituierten Aminopolyorganosiloxane ($S_H$) können wie in WO 02/092666 beschrieben durch Einführung der Reste (ε) und gegebenenfalls eines oder mehrerer der weiteren Substituenten, in entsprechende Ausgangsaminopolyorganosiloxane (S), die primäre und/oder sekundäre Aminogruppen enthalten, die über Alkylenbrücken oder Mono - oder Oligo-(alkylenamino)-alkylenbrücken an Siliciumatome des Polysiloxangrundgerüstes gebunden sind, hergestellt werden. Dabei werden die Ausgangsaminopolyorganosiloxane (S) mit mindestens einem Alkoholpolyglykoläther-monoglycidyläther (H) umsetzt und gegebenenfalls anschliessend acyliert und/oder alkyliert und/oder benzyliert und/oder protoniert.

Die Alkoholpolyglykoläther-monoglycidyläther (H) sind im allgemeinen Glycidyläther von Alkoholpolyglykoläthern und können durch die folgende Formel dargestellt werden

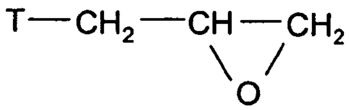 (I)

worin T den Rest des entsprechenden tensidischen Alkoholpolyglykoläthers T-H bedeutet, insbesondere wie sie durch Glycidylätherbildung eines entsprechenden tensidischen Alkoholpolyglykoläthers T-H herstellbar sind.

Die Alkoholpolyglykoläther T-H sind solche der folgenden durchschnittlichen Formel

$$R_1 \text{-}(O\text{-}X)_q\text{-} OH \qquad (II),$$

worin

$R_1$ einen alkylaromatischen oder aliphatischen Kohlenwasserstoffrest mit 9 bis 24 Kohlenstoffatomen,
X $C_{2-4}$-Alkylen

und

q 4 bis 50,

bedeuten, wobei mindestens 80 % der q Alkylenoxygruppen Äthylenoxygruppen sind. Die Kohlenwasserstoffreste $R_1$ sind alkylaromatisch oder aliphatisch und enthalten vorteilhaft 9 bis 22, vorzugsweise 9 bis 18, besonders bevorzugt 11 bis 16 Kohlenstoffatome.

Wenn $R_1$ aliphatisch ist, ist er bevorzugt gesättigt; $R_1O$- steht in diesem Fall vorzugsweise für den Rest eines primären, gesättigten, aliphatischen Alkohols bzw. eines primären Alkanols, welches vorteilhaft 9 bis 18, vorzugsweise 11 bis 16 Kohlenstoffatome enthält. Der entsprechende aliphatische Alkohol $R_1OH$ kann ein linearer Fettalkohol sein, z.B. Laurylalkohol, Myristylalkohol, Cetylalkohol, Stearylalkohol oder Behenylalkohol oder auch ein Synthesealkohol sein (z.B. aus der Oxosynthese oder aus der Ziegler-Synthese), welcher linear oder verzweigt sein kann, z.B. Nonanol, Isononol, Decanol, Isodecanol, Undecanol, Tridecanol, Isotridecanol oder Isohexadecanol.

Die Anzahl q der an diesen Alkohol zur Bildung des Alkoholpolyglykoläthers der Formel II angelagerten Alkylenoxyeinheiten ist vorteilhaft im Bereich von 4 bis 30, vorzugsweise 4 bis 20. Mindestens 80 % der q Alkylenoxyeinheiten sind Äthylenoxyeinheiten, vorzugsweise sind 100 % der Alkylenoxyeinheiten Äthylenoxyeinheiten.

Die Anzahl Äthylenoxyeinheiten in T-H ist vorteilhaft 4 bis 30, vorzugsweise 4 bis 18, besonders bevorzugt 5 bis 12.

Die Tenside T-H der Formel (II) sind vorteilhaft solche, deren HLB größer als 7 ist, und vorteilhaft im Bereich von 7 bis 17, vorzugsweise 8 bis 16,5, besonders bevorzugt 9 bis 16, liegt.

Die primären und/oder sekundären Aminogruppen in den mit (H) umzusetzenden Aminopolysiloxanen (S) sind insbesondere Teil der Si-gebundenen Aminoalkylgruppen oder Amino-mono- oder -oligo-(alkylenamino)-alkyl-Gruppen und können solche sein, wie sie üblicherweise in sonst nicht weitermodifizierten Aminopolysiloxanen vorkommen und durch Verwendung entsprechender Monomeren bei der Herstellung der jeweiligen Aminopolysiloxane entstehen können. Ihre Alkyl- und Alkylengruppen sind vorteilhaft solche mit 2 bis 4 Kohlenstoffatomen und können linear oder, wenn sie 3 oder 4 Kohlenstoffatome enthalten, auch verzweigt sein. Vorzugsweise enthalten die an Si gebundenen Aminoalkylgruppen 3 oder 4 Kohlenstoffatome im Alkylrest; die zwei Aminogruppen verbindenden Alkylengruppen enthalten vorzugsweise 2 oder 3 Kohlenstoffatome. Vornehmlich sind die primären Aminogruppen und die gegebenenfalls vorhandenen sekundären Aminogruppen in (S) Bestandteile von an Siliciumatome des Polysiloxangrundgerüstes gebundenen Aminoalkyl-Gruppen oder Amino-mono- oder -oligo-(alkylenamino)-alkyl-Gruppen der Formel

$$H_2N\text{---}(Y_2\text{---}NH)_p\text{---}Y_1\text{---} \qquad (\alpha),$$

worin

$Y_1$ Propylen-1,2 oder -1,3 oder 2-Methyl-propylen-1,3,
$Y_2$ Äthylen oder Propylen

und

p 0, 1 oder 2

bedeuten.

$Y_1$ steht vorteilhaft für 2-Methyl-propylen-1,3 oder vorzugsweise für Propylen-1,3; $Y_2$ steht insbesondere für Propylen-1,2 oder -1,3 oder vorzugsweise für Äthylen; p steht vorteilhaft für 0 oder 1, vorzugsweise für 1.

Vorzugsweise sind die Reste der Formel (α) solche der Formel

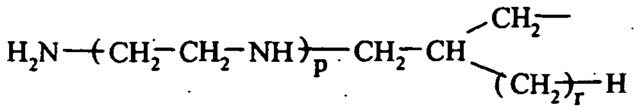

(α'),

worin r 0 oder 1 bedeutet,
besonders bevorzugt der Formel

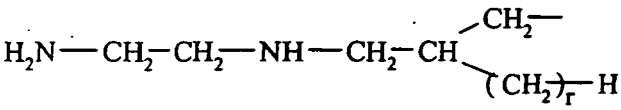

(α").

Der Index r steht besonders bevorzugt für 0.

Durch die Umsetzung mit (H) werden die jeweiligen Reste (ε) bzw.

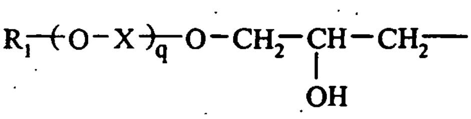

(ε'),

in die Aminoalkylgruppen oder Amino-mono- oder -oligo-(alkylenamino)-alkylGruppen, insbesondere in diejenigen der Formel (α), eingeführt. Dabei reagiert die primäre Aminogruppe vorrangig, so daß ein erster Rest (ε) ein Wasserstoffatom der primären Aminogruppe ersetzt, und weitere Reste (ε) in statistischer Verteilung Wasserstoffatome der vorhandenen sekundären Aminogruppen ersetzen. Gewünschtenfalls können noch acylierbare Aminogruppen acyliert werden und/oder gegebenenfalls vorhandene alkylierbare Aminogruppen alkyliert und/oder benzyliert werden.

Durch die Acylierung werden vorzugsweise niedrigmolekulare Acylreste eingeführt, vorteilhaft solche mit 2 bis 4 Kohlenstoffatomen, z.B. Acetyl, Propionyl oder Butyryl, worunter Acetyl besonders bevorzugt ist. Durch die Alkylierung bzw. Benzylierung können ebenfalls vorzugsweise niedrigmolekulare Alkylreste eingeführt werden, vorteilhaft Alkylreste mit 1-4 Kohlenstoffatomen, vorzugsweise Äthyl oder Methyl, bzw. Benzylreste.

Die Umsetzung von (S) mit (H) wird vorteilhaft so geführt, daß praktisch alle primären Aminogruppen von (S) mit (H) so umgesetzt werden, daß sie mindestens monosubstituiert sind. Von den dann verbleibenden sekundären Aminogruppen werden mindestens soviele Wasserstoffatome durch einen Rest (ε) ersetzt, dass der erforderliche Substitutionsgrad von durchschnittlich mindestens 1,5, vornehmlich mindestens 1,8, vorzugsweise mindestens 2 Resten der Formel (ε) pro Sigebundener Aminoalkylgruppe oder Amino-mono- oder -oligo-(alkylenamino)-alkylGruppe erreicht wird. Der Anteil an sekundären Aminogruppen die mit (H) umgesetzt werden, kann je nach Anzahl sekundärer Aminogruppen in dieser Si-gebundenen Gruppe, insbesondere je nach der Bedeutung von p im Rest der Formel (α) bzw. (α'), variieren. Bei p = 0 beträgt dieser Anteil insbesondere mindestens die Hälfte, bzw. 50 bis 100 % der sekundären Aminogruppen, vorteilhaft 80 bis 100 %, vorzugsweise 95 bis 100 % davon; bei p = 1, mindestens ¼, insbesondere 25 bis 100 % der sekundären Aminogruppen, vorteilhaft 50 bis 100 %, vorzugsweise 80 bis 100 % davon; bei p = 2, mindestens $^1/_6$, insbesondere 16,7 bis 100 % der sekundären Aminogruppen, vorteilhaft 40 bis 100 %, vorzugsweise 60 bis 100 % davon.

Die Umsetzung von (S) mit (H) kann z.B. bei p ≥ 1 vorteilhaft bis zu einem Substitutionsgrad im Bereich von 40 bis 100 %, vorzugsweise 45 bis 100 %, besonders 50 bis 100 %, bei p = 0 vorteilhaft bis zu einem Substitutionsgrad im Bereich von 75 bis 100 %, vorzugsweise 80 bis 100 %, besonders 90 bis 100 %, [bezogen auf die reaktionsfähigen Wasserstoffatome der basischen Aminogruppen in (S)] durchgeführt werden.

In einer weiteren bevorzugten Ausführungsform der Erfindung weist das substituierte Aminopolyorganosiloxan ($S_H$) einen (ε)-Substituitionsgrad der gesamten Aminogruppen im Bereich von 40 bis 100 %, bevorzugt im Bereich von

50 bis 100 % und besonders bevorzugt im Bereich von 60 bis 100 % auf bzw. sind die gesamten Aminogruppen des substituierten Aminopolyorganosiloxans ($S_H$) zu 40 bis 100 %, bevorzugt zu 50 bis 100 % und besonders bevorzugt zu 60 bis 100 % durch Reste der Formel ($\varepsilon$) substituiert.

Die nach der Umsetzung von (S) mit (H) übrigen reaktionsfähigen Wasserstoffatome der basischen Aminogruppen können gegebenenfalls mindestens teilweise (z.B. 5 bis 100 %, insbesondere 10 bis 90 %) mittels Acylierung durch Acylreste aliphatischer Monocarbonsäuren, vorzugsweise solchen mit 2-4 Kohlenstoffatomen, ersetzt werden oder mittels Alkylierung bzw. Benzylierung durch Methyl oder Äthyl oder Benzyl ersetzt werden. Die Alkylierung und/oder Benzylierung kann, je nach vorhandenen Aminogruppen und eingesetzten Alkyierungs- bzw. Benzylierungsmitteln, gegebenenfalls zu entsprechenden sekundären oder tertiären Aminogruppen oder bis zur quaternären Ammoniumstufe führen. Basische Aminogruppen die nicht quaterniert werden, können gegebenenfalls protoniert werden.

Die bevorzugten aus den Si-gebundenen Aminoalkylgruppen oder Amino-mono- oder -oligo-(alkylenamino)-alkyl-Gruppen, insbesondere aus den Si-gebundenen Gruppen ($\alpha$), stammenden, mit (H) umgesetzten und gegebenenfalls acylierten und/oder gegebenenfalls weiter alkylierten und/oder benzylierten Gruppen, können durch die folgende durchschnittliche Formel dargestellt werden

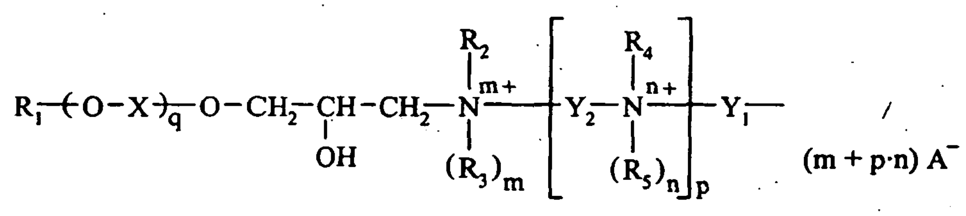

($\beta$),

worin

m 0 oder 1,
n 0 bis 1,
$R_2$ Wasserstoff, $C_{1-2}$-Alkyl, Benzyl, oder einen Rest der Formel ($\varepsilon$') oder, wenn m = 0 ist, auch einen Rest der Formel $R_6$. CO - ,
$R_3$ Wasserstoff oder, wenn $R_2$ für $C_{1-2}$-Alkyl, Benzyl oder einen Rest der Formel ($\varepsilon$') steht, auch $C_{1-2}$-Alkyl oder Benzyl,
$R_4$ Wasserstoff, $C_{1-2}$-Alkyl, Benzyl, einen Rest der Formel ($\varepsilon$') oder, wenn n = 0 ist, auch einen Rest der Formel $R_6$ -CO-,
$R_5$ Wasserstoff oder, wenn $R_4$ für $C_{1-2}$-Alkyl oder einen Rest der Formel ($\varepsilon$') steht, auch $C_{1-2}$-Alkyl oder Benzyl,
$R_6$ $C_{1-3}$-Alkyl
und $A^-$ ein einwertiges Anion

bedeuten, mit den Maßgaben dass
mindestens 50 % der q Alkylengruppen in der Bedeutung von X Äthylen bedeuten und die Reste der Formel ($\beta$) durchschnittlich mindestens 1,5 Reste der Formel ($\varepsilon$') pro Rest der Formel ($\beta$) enthalten.

Bevorzugte, aus den Gruppen der Formeln ($\alpha$') bzw. ($\alpha$") stammende Gruppen können durch die folgenden durchschnittlichen Formeln

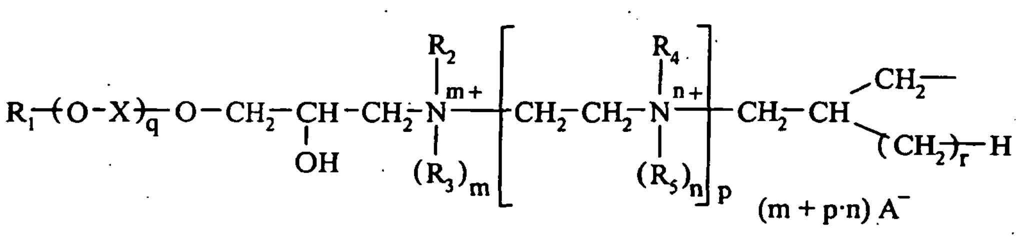

($\beta$')

und

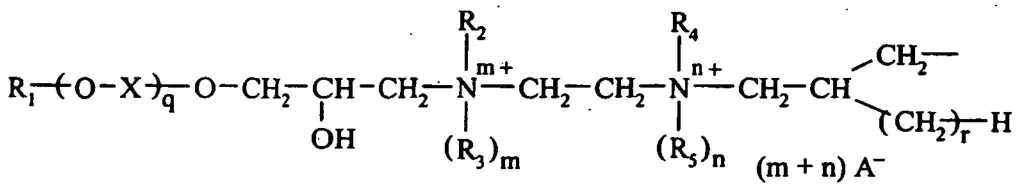 (β'')

dargestellt werden, wobei vorzugsweise mindestens eines von $R_2$ und $R_4$ auch für einen Rest der Formel (ε') steht.

Darunter sind auch die nicht quaternierten, besonders diejenigen der Formel

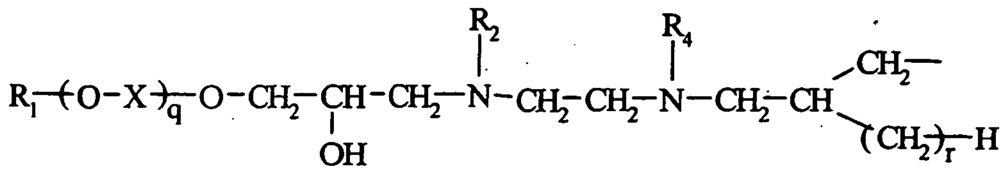 (β''')

und deren protonierten Derivate, bevorzugt.

Die aus den Si-gebundenen Aminoalkylgruppen oder Amino-mono- oder -oligo-(alkylen-amino)-alkyl-Gruppen insbesondere der Formel (α), durch die oben beschriebene Umsetzung hergestellten substituierten Derivate, insbesondere der Formel (β), enthalten die jeweiligen Substituenten in herstellungsentsprechender Verteilung. So können die bevorzugten aus den Resten der Formel (α'') stammenden Gruppen (β'') hauptsächlich durch die folgenden Formeln dargestellt werden:

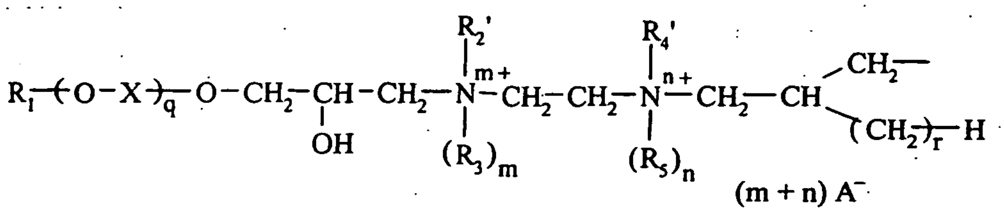 ($β_1$),

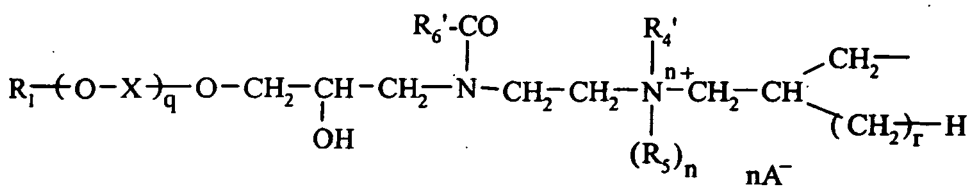 ($β_2$),

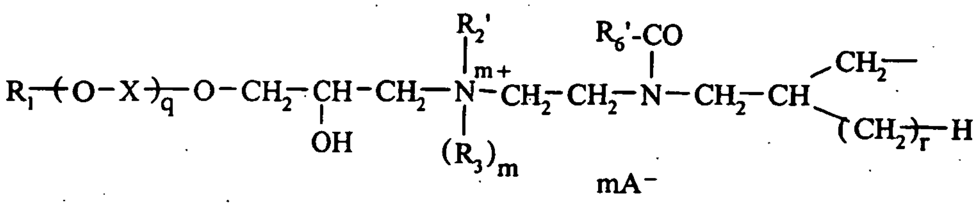 ($β_3$),

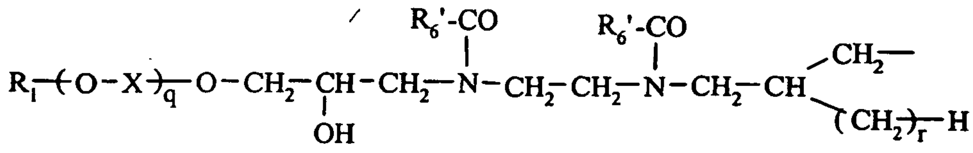

(β4),

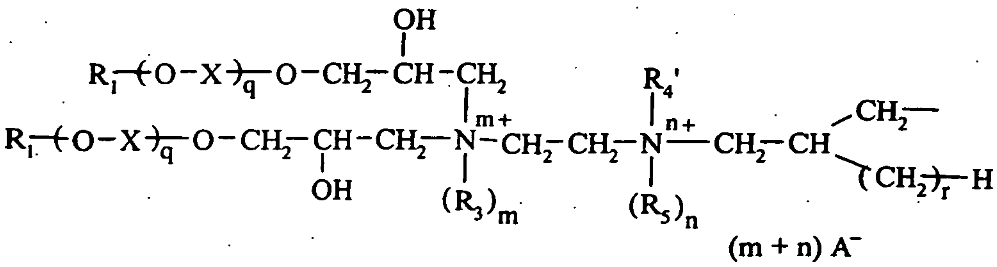

(β5),

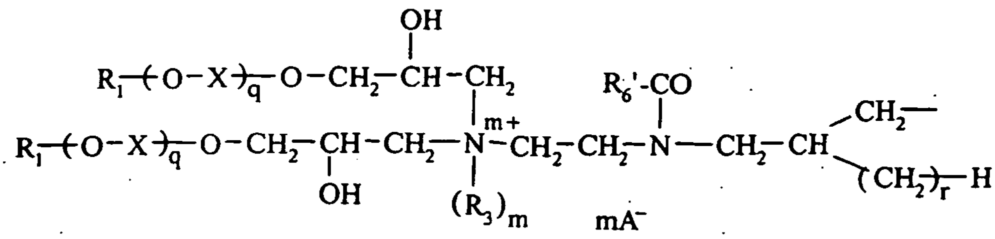

(β6),

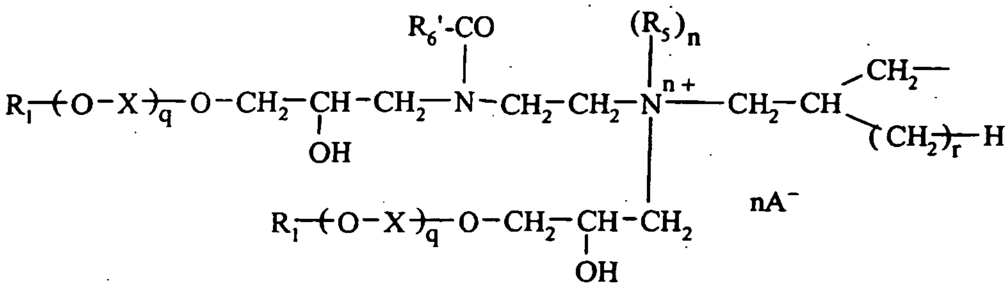

(β7),

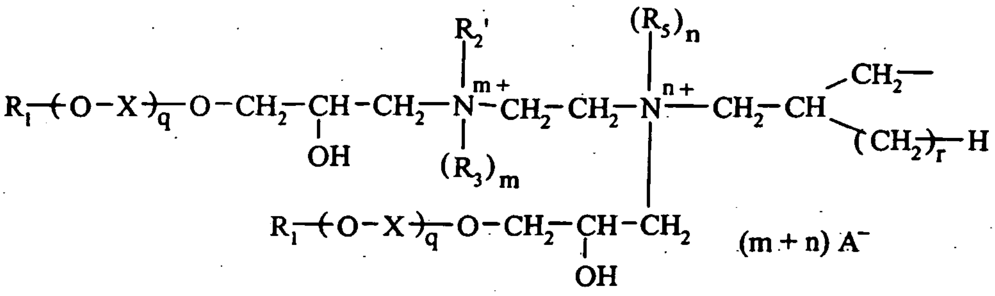

(β8)

und/oder

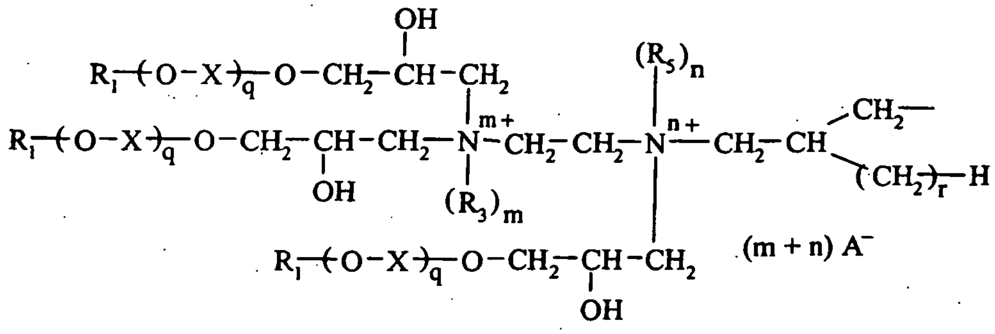

(β₉),

worin $R_2$' Wasserstoff, Methyl, Äthyl oder Benzyl,
$R_4$' Wasserstoff, Methyl, Äthyl oder Benzyl und $R_6$' Methyl oder Äthyl
bedeuten.

In den erschöpfend oder nahezu erschöpfend mit (H) umgesetzten Aminopolysiloxanen (S) überwiegen entsprechend diejenigen, welche (p+2) Reste der Formel (ε), vorzugsweise (ε'), enthalten, unter denjenigen der obigen Formeln ($\beta_1$) bis ($\beta_9$) also diejenigen der Formel ($\beta_9$), und können von entsprechend kleineren Anteilen an niedriger durch (ε) bzw. (ε') substituierten begleitet sein, besonders solchen der Formeln ($\beta_5$) und/oder ($\beta_8$).

In den zu einem niedrigeren Umsetzungsgrad mit (H) umgesetzten, z.B. in denjenigen in welchen 50 bis 75 % der ersetzbaren stickstoffgebundenen Wasserstoffatome von (α), besonders worin p 1 oder 2 bedeutet, vorzugsweise von (α') bzw. (α"), durch Reste der Formel (ε) bzw. (ε') ersetzt sind und die übrigen gegebenenfalls acyliert und/oder alkyliert und/oder benzyliert sind, überwiegen entsprechend diejenigen, welche 2 bis (p+1) Reste der Formel (ε), vorzugsweise (ε'), enthalten, unter denjenigen der obigen Formeln ($\beta_1$) bis ($\beta_9$) also diejenigen der Formeln ($\beta_5$), ($\beta_6$), ($\beta_7$) und/oder ($\beta_8$), neben kleineren Anteilen an ($\beta_9$) und/oder ($\beta_1$), ($\beta_2$), ($\beta_3$) und/oder ($\beta_4$).

Die aus Si-gebundenen Aminoalkylresten, besonders aus den Resten der Formel

$$H_2N - Y_1 - \quad (\alpha''')$$

insbesondere

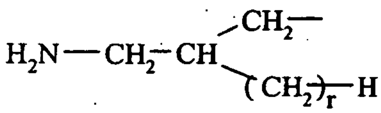

(α"")

stammenden sind vorteilhaft erschöpfend oder nahezu erschöpfend mit (H) umgesetzt, sodass die mit Resten (ε) bzw. (ε') disubstituierten überwiegen, bzw. in den bevorzugten aus (α"") stammenden, hauptsächlich die Reste der Formel

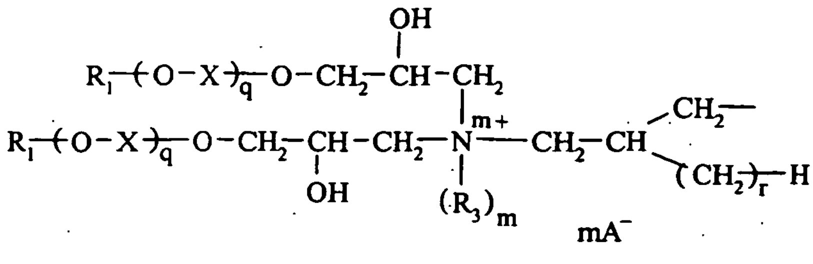

(β₁₀),

überwiegen, und im Produkt verhältnismäßig kleinere Anteile an durch Reste der Formel (ε) bzw. (ε') monosubstituierten gegebenenfalls vorkommen können, insbesondere, in den bevorzugten aus (α"") stammenden, hauptsächlich die Reste der Formel

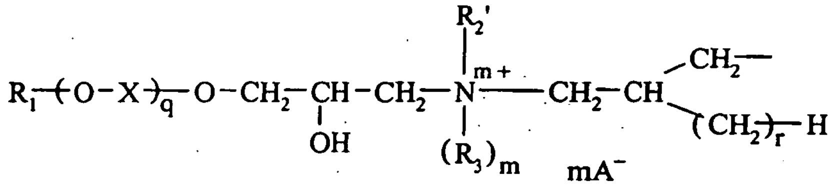 (β11).

Als Ausgangspolysiloxane (S) eignen sich beliebige aminosubstituierte Polysiloxane, die entsprechende Si-gebundene Aminoalkylgruppen bzw. Amino-mono- oder -oligo-(alkylenamino)-alkyl-Gruppen enthalten. Es eignen sich im allgemeinen beliebige entsprechende Aminopolysiloxane mit polykationischem bzw. polybasischem Charakter, im wesentlichen solche, die aus wiederkehrenden Dimethylsiloxyeinheiten und Aminosiloxyeinheiten aufgebaut sind. Sie können einen linearen Aufbau oder auch einen verzweigten und/oder vernetzten Aufbau (z.B. einfach oder mehrfach verzweigt oder vernetzt) aufweisen. Die Endgruppen können einen reaktiven Substituenten, insbesondere z.B. Hydroxy oder Alkoxy, enthalten oder gegebenenfalls auch blockiert sein; z.B. mit Trimethylsiloxy. Nach einer weiteren Variante können die Endgruppen auch die oben genannten Aminoalkylgruppen bzw. Amino-mono- oder -oligo-(alkylenamino)-alkyl-Gruppen enthalten.

Vorzugsweise sind die Aminopolysiloxane (S) aus wiederkehrenden Einheiten der folgenden Formeln aufgebaut:

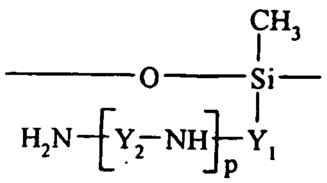 (γ1)

und

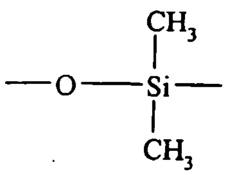 (γ2).

Die Endgruppen entsprechen vorzugsweise den Formeln:

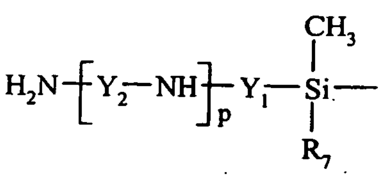 (γ3)

und/oder

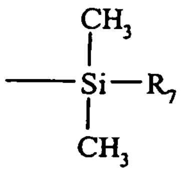 ($\gamma_4$),

worin $R_7$ Methyl, Hydroxy, Methoxy oder Ethoxy bedeutet.

Gegebenenfalls kann (S) auch Si-verzweigte Einheiten der Formel

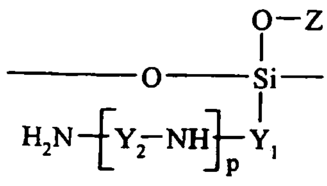 ($\gamma_5$)

enthalten,

worin Z einen Si-gebundenen (Poly)siloxan- oder Silylrest bedeutet, der eine oder mehrere der Gruppen der Formel ($\gamma_1$), ($\gamma_2$), ($\gamma_3$) und/oder ($\gamma_4$) sowie gegebenenfalls weitere solche Si-Verzweigungen bzw. Vernetzungen (z.B. einfach oder mehrfach verzweigt oder/und vernetzt) enthält.

Die Aminopolyorganosiloxane (S) können durch an sich gebräuchliche typische Kennwerte gekennzeichnet werden, z.B. durch ihr durchschnittliches Molekulargewicht und den Gehalt an Aminstickstoff, sowie auch durch ihre Viskosität Das durchschnittliche Molekulargewicht und der Gehalt an Aminstickstoff der Aminopolyorganosiloxane (S) können in weiten Bereichen schwanken, wobei zum Zwecke der Erfindung diejenigen mit einer niedrigen Aminzahl vornehmlich geeignet sind, besonders diejenigen mit einer Aminzahl $\leq 3$.

Vorteilhaft weisen die Aminopolysiloxane (S) eine Viskosität im Bereich von 500-30000, vornehmlich 200-20000, vorzugsweise 300-3000 cP auf (Brookfield Rotationsviskosimeter RV, Spindel Nr. 5, 20°C). Die Aminzahl von (S) liegt vorteilhaft im Bereich von 0,05 bis 3, vorzugsweise 0,1 bis 2, besonders bevorzugt 0,15 bis 1.

Schematisch können die aus den oben genannten Einheiten bestehenden Aminopolysiloxane (S) insbesondere durch die folgende durchschnittliche Sammelformel dargestellt werden:

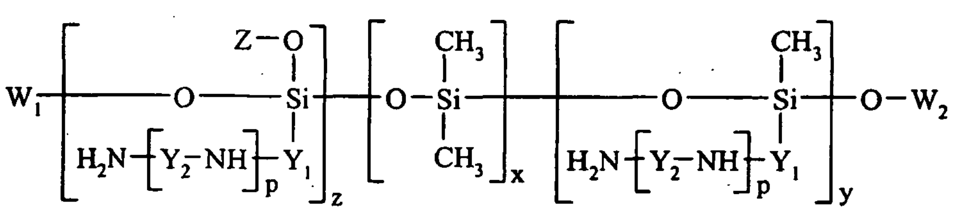 (III),

worin $W_1$ und $W_2$ jeweils eine Gruppe der Formel ($\gamma_3$) oder ($\gamma_4$) bedeuten, das Molekül mindestens eine Gruppe der Formel ($\alpha$) bzw. ($\gamma_1$), ($\gamma_3$) und/oder ($\gamma_5$) aufweist und die Indizes x, y und z so gewählt werden, dass das Polymere die oben angegebenen Werte für Aminzahl, Viskosität und Molekulargewicht aufweist. [Die obige Formel (III) dient zur Veranschaulichung der vorkommenden Monomereinheiten und deren Anzahl, nicht aber ihrer Verteilung oder Stellung im Polymermolekül]. Das Verhältnis der Anzahl Dimethylsiloxyeinheiten zur Anzahl Aminoalkyl-siloxyeinheiten und/oder Amino-mono- oder -oligo-(alkylenamino)-alkyl-siloxyeinheiten, insbesondere der Formel

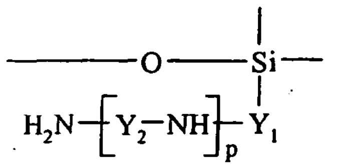

($\gamma_6$)

liegt vorteilhaft im Bereich von 3/1 bis 600/1, vorzugsweise 10/1 bis 200/1.

Zur Copolymerisation werden vorzugsweise die aminogruppenhaltigen Silane mit $\alpha,\omega$-Dihydroxy-polydimethylsiloxan, vorteilhaft mit einem durchschnittlichen Molekulargewicht

$$\overline{M_W}$$

$_w$ im Bereich von 500 bis 10000, vorzugsweise 1000 bis 7000, oder mit zyklischen Siloxanen, z.B. Hexamethylcyclotrisiloxan, Octamethylcyclotetrasiloxan, Decamethylcyclopentasiloxan, Dodecamethylcyclohexasiloxan und technischen Gemischen von zwei oder mehreren davon, copolymerisiert. Als Silane kommen vornehmlich an Si aminoalkylsubstituierte oder amino-mono- oder -oligo-(alkylenamino)-alkylsubstituierte Trimethoxy- oder -ethoxysilane oder Dimethoxy- oder -ethoxymethylsilane in Betracht, worin die Si-gebundene Aminoalkylgruppe oder Amino-mono- oder -oligo-(alkylenamino)-alkyl-Gruppe vornehmlich der Formel ($\alpha$), vorzugsweise ($\alpha$'), besonders ($\alpha$"), entspricht.

Wird ein aminogruppenhaltiges Trimethoxysilan zur Einführung der Einheiten der Formel ($\gamma_1$) verwendet, kann je nach Umsetzungsbedingungen die Methoxygruppe zur Hydroxygruppe verseift werden oder an dieser Stelle eine Verzweigung des Copolymeren erfolgen, wie mit der Formel ($\gamma_5$) gezeigt.

Je nach gewählten Herstellungsbedingungen können die aminogruppenhaltigen Einheiten im Molekül - z.B. im Molekül der Formel (III) - statistisch verteilt sein oder endständig sein oder wie in Blockpolymeren gruppiert sein oder noch sich gegen die Extremitäten der linearen Ketten anhäufen.

Für die Herstellung der erfindungsgemäß in kosmetischen und pharmazeutischen Mitteln eingesetzten Polysiloxane ($S_H$) sind diejenigen Polysiloxane (S) bevorzugt, welche einen gegebenenfalls verzweigten, vorwiegend linearen Aufbau des Polysiloxangrundgesetzes aufweisen, worin die Einheiten der Formel ($\gamma_2$) neben Einheiten der Formel ($\gamma_1$) überwiegen.

Bevorzugt werden Polysiloxane, worin die vorhandenen Si-gebundenen Aminoalkyl-Gruppen oder Amino-mono- oder -oligo-(alkylenamino)-alkyl-Gruppen, insbesondere die Gruppen der Formel ($\alpha$) bzw. ($\alpha$') oder ($\alpha$"), durch Einführung der Reste ($\varepsilon$) bzw. ($\varepsilon$') und gegebenenfalls weiterer Substituenten, wie in WO 02/092666 beschrieben, am Stickstoff entsprechend substituiert sind. Die Polysiloxane enthalten insbesondere wiederkehrende Einheiten der Formeln

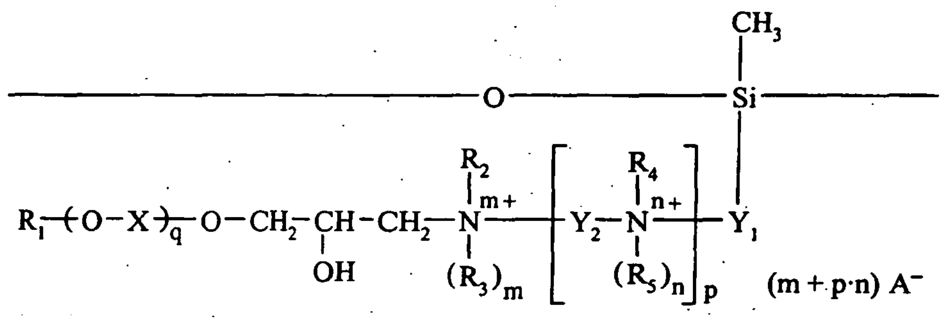

($\gamma_7$),

und ($\gamma_2$), und endständige, sauerstoffgebundene Silylgruppen der Formel ($\gamma_3$) und/oder

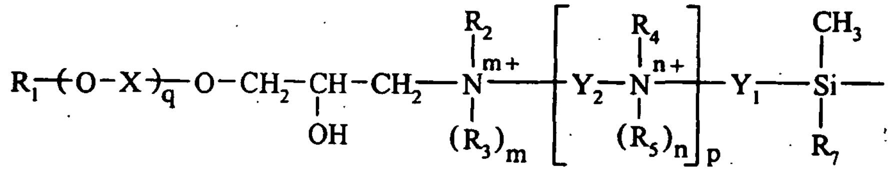

(γ8)

$(m + p \cdot n)\ A^-$

und, wenn die Ausgangspolysiloxane (S) Verzweigungen, insbesondere wie in der Formel ($\gamma_5$), enthalten, auch entsprechend verzweigte Gruppen, insbesondere solche der Formel

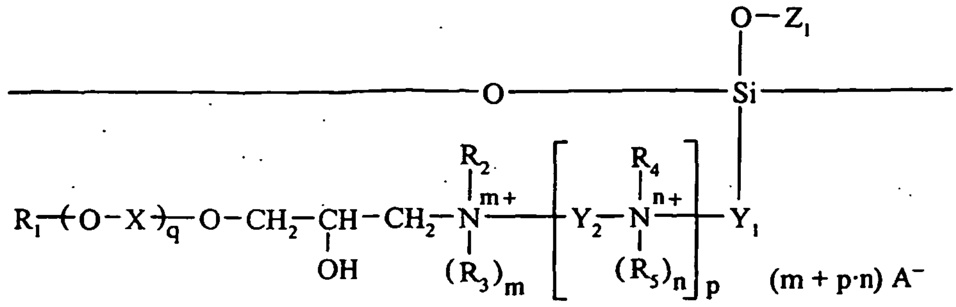

(γ9)

worin $Z_1$ einen Si-gebundenen (Poly)siloxan- oder Silylrest bedeutet, der eine oder mehrere der Gruppen der Formel ($\gamma_2$), ($\gamma_4$), ($\gamma_7$) und/oder ($\gamma_8$) sowie gegebenenfalls weitere solche Si-Verzweigungen enthält,
mit der Bedingung, dass im Molekül durchschnittlich mindestens 1,5, vorteilhaft mindestens 1,8, vorzugsweise mindestens zwei Reste der Formel (ε') pro insgesamt vorkommende Si-gebundene Aminoalkyl- oder Amino-mono- oder -oligo-(alkylenamino)-alkyl-gruppe der Formeln (β) vorhanden sind.
Wenn in den oben beschriebenen Polysiloxanen in den Resten der Formel (β) m und/oder n mindestens teilweise gleich null sind und der entsprechende Substituent $R_2$ bzw. $R_4$ nicht für einen Acylrest $R_6$-CO- steht, können diese Reste bzw. die Polysiloxane gewünschtenfalls protoniert sein.

Das durchschnittliche Molekulargewicht der erfindungsgemäß in kosmetischen und pharmazeutischen Mitteln eingesetzten substituierten Aminopolyorganosiloxane ($S_H$), kann in einem breiten Bereich variieren, z.B. je nach gewählten Ausgangsprodukten, Mengenverhältnissen der Reagenzien und Reaktionsbedingungen, insbesondere Polymerisations- und Substitutionsbedingungen, z.B. im Bereich von 15.000 bis 2.000.000, vorteilhaft von 30.000 bis 1.750.000, vorzugsweise von 50.000 bis 1.500.000. Der Stickstoffgehalt von ($S_H$) - insbesondere stammend aus den Aminogruppen in (S) durch Umsetzung mit (H) und gegebenenfalls weitere Substitution zu substituierten Amino- und/oder Ammoniumgruppen und gegebenenfalls Amidgruppen und mitumfassend auch gegebenenfalls übrige nicht umgesetzte Aminogruppen - ist vorzugsweise niedrig und liegt vorteilhaft im Bereich von 0,03 bis 4,2 Gew.-%, vorteilhaft im Bereich von 0,1 bis 2,8 Gew.-% und vorzugsweise im Bereich von 0,16 bis 1,4 Gew.-%.

Die in den kosmetischen oder pharmazeutischen Mitteln eingesetzten Aminopolyorganosiloxane ($S_H$) haben eine ausgeprägte Hydrophilie, die durch Einbau entsprechender Gruppen und Substituenten modifiziert werden kann. Darüber hinaus wirken die oben beschriebenen Aminopolyorganosiloxane ($S_H$) selbstemulgierend und sind kompatibel mit lipophilen Komponenten und Ölen.

Bevorzugte Ausführungsformen der Mittel sind Fluids, Gele, Öle, Schäume, Sprays, Lotions, Cremegels, Cremes und Puder.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-, Heiß-, Heiß/Kalt- bzw. PIT-Emulgierung erfolgen.

Gute Substantivität, konditionierende Wirkung, sowie glanz- und volumengebende Effekte der oben beschriebenen Aminopolyorganosiloxane ($S_H$) macht man sich zur Herstellung von Haarbehandlungsmitteln, vorzugsweise Schampoos, Haarconditioner, Haarkuren, Stylingmittel, Haarspülungen, Volumenspray, Styling-Fluid, Haarschaum, Haargel, Festiger, Haarspray, Mousse, Haarölen und Spitzenfluids zu Nutze.

Aminopolyorganosiloxane ($S_H$) verbessern das Farbaufziehverhalten von Haarfärbemittel und sind somit wertvolle Bestandteile in Haartönungs- und -färbemitteln. Gleichzeitig verbessern sie zusätzlich als Farbschutzadditive die Haltbarkeit von Haartönungen oder permanten Haarfärbemitteln.

Gegenstand der Erfindung ist die Verwendung eines beschriebenen kosmetischen oder pharmazeutischen Mittels zum Schutz und zur Erhaltung der Farbe in gefärbten Keratinfasern, vorzugsweise in gefärbtem menschlichem Haar. Vorzugsweise enthält das Mittel bei dieser Verwendung von 0,01 bis 10 Gew.-% bezogen auf das fertige Mittel an substituiertem Aminopolyorganosiloxan ($S_H$).

In einer weiteren bevorzugten Ausführungsform der Erfindung handelt es sich bei den kosmetischen oder pharmazeutischen Mitteln um Rinse-off Produkte, insbesondere um Duschbäder, Duschgels oder Schaumbäder.

In einer weiteren bevorzugten Ausführungsform der Erfindung handelt es sich bei den kosmetischen oder pharmazeutischen Mitteln um Leave-on Produkte.

In einer weiteren bevorzugten Ausführungsform der Erfindung handelt es sich bei den kosmetischen und pharmazeutischen Mitteln um tensidfreie Mittel, insbesondere um tensidfreie feste Mittel oder um tensidfreie Emulsionen.

In einer weiteren bevorzugten Ausführungsform der Erfindung handelt es sich bei den kosmetischen oder pharmazeutischen Mitteln um Additive für Dauerwellenmittel, insbesondere um Conditioner.

Die kosmetischen oder pharmazeutischen Mittel auf wässriger oder wässrig-alkoholischer Basis enthalten Aminopolyorganosiloxane ($S_H$) vorzugsweise in den Gewichtsmengen von 0,01 bis 30 %, besonders bevorzugt von 0,2 bis 10 %, insbesondere bevorzugt von 0,5 bis 2 % bezogen auf die fertigen Mittel.

Die kosmetischen oder pharmazeutischen Mittel in wasserfreier Form auf Basis von Ölen enthalten Aminopolyorganosiloxane ($S_H$) vorzugsweise in den Gewichtsmengen von 0,01 bis 80 %, besonders bevorzugt von 0,05 bis 60 %, insbesondere bevorzugt von 0,1 bis 50 % bezogen auf die fertigen Mittel.

Die kosmetischen oder pharmazeutischen Mittel in Form einer Emulsion enthalten substituierte Aminopolyorganosiloxane ($S_H$) vorzugsweise in Gewichtsmengen von 0,01 bis 30 %, besonders bevorzugt von 0,05 bis 10 % und insbesondere bevorzugt von 0,1 bis 5 % bezogen auf das fertige Mittel.

In einer weiteren bevorzugten Ausführungsform sind die Mittel Öl-in-Wasser Emulsionen mit einem Wasseranteil von 5 bis 95 Gew.-%, bevorzugt 15 bis 75 Gew.-%, besonders bevorzugt 25 bis 85 Gew.-%.

In einer weiteren bevorzugten Ausführungsform sind die Mittel Wasser-in-Öl Emulsionen mit einem Ölanteil von 5 bis 95 Gew.-%, bevorzugt 15 bis 75 Gew.-%, besonders bevorzugt 25 bis 65 Gew.-%.

Für die Mittel auf wässrig-alkoholischer oder alkoholischer Basis kommen alle ein - oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Die Mittel auf Ölbasis können vorzugsweise enthalten: Kohlenwasserstofföle mit linearen oder verzweigten, gesättigten oder ungesättigten $C_7$-$C_{40}$-Kohlenstoffketten, beispielsweise Dodecan, Isododecan, Cholesterol, hydrierte Polyisobutylene, Docosane, Hexadecan, Isohexadecan, Paraffine und Isoparaffine, aber auch Triglyceride tierischen und pflanzlichen Ursprungs, beispielweise Rindertalg, Schweineschmalz, Gänseschmalz, Perhydrosqualen, Lanolin, Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Olivenöl, Erdnussöl, Rapsöl und Kokosnussöl und synthetische Öle wie Purcellinöl, lineare und/oder verzweigte Fettalkohole und Fettsäureester, bevorzugt Guerbetalkohole mit 6 bis 18, vorzugsweise 8 bis 10, Kohlenstoffatomen; Ester von linearen ($C_6$-$C_{13}$)-Fettsäuren mit linearen ($C_6$-$C_{20}$)-Fettalkoholen; Ester von verzweigten ($C_6$-$C_{13}$)-Carbonsäuren mit linearen ($C_6$-$C_{20}$)-Fettalkoholen, Ester von linearen ($C_6$-$C_{18}$)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Alkoholester von $C_1$-$C_{10}$-Carbonsäuren oder $C_2$-$C_{30}$-Dicarbonsäuren, Ester wie Dioctyladipat, Diisopropyl dimer dilineloat; Propylenglycole/-dicaprilat oder Wachse wie Bienenwachs, Paraffinwachs oder Mikrowachse, gegebenenfalls in Kombination mit hydrophilen Wachsen, wie z.B. Cetylstearylalkohol; Fluorierte und perfluorierte Öle;

Monoglyceride von $C_1$-$C_{30}$-Carbonsäuren, Diglyceride von $C_1$-$C_{30}$-Carbonsäuren, Triglyceride von $C_1$-$C_{30}$-Carbonsäuren, beispielsweise Triglyceride der Capryl/Caprinsäuren, Ethylenglykolmonoester von $C_1$-$C_{30}$-Carbonsäuren, Ethylenglycoldiester von $C_1$-$C_{30}$-Carbonsäuren, Propylenglycolmonoester von $C_1$-$C_{30}$-Carbonsäuren, Propylenglycoldiester von $C_1$-$C_{30}$-Carbonsäuren, sowie propoxilierte und ethoxilierte Derivate der oben genannten Verbindungsklassen. Die Carbonsäuren können lineare oder verzweigte Alkylgruppen oder aromatische Gruppen enthalten. Beispielhaft genannt seien Diisopropylsebacat, Diisopropyladipate, Isopropylmyristat, Isopropylpalmitat, Myristylpropionat, Ethylenglycoldistearat, 2-Ethylhexylpalmitat, Isodecylneopentanoat, Di-2-Ethylhexylmaleat, Cetylpalmitat, Myristylmyristat, Stearylstearat, Cetylstearat, Behenylbehenat, Dioctylmaleate, Dioctylsebacat, Cetyloctanoat, Diisopropyl dilinoleate, Caprylic/ Capryltriglycerid, PEG-6-Caprylic/ Capryltriglycerid, PEG-8-Caprylic/ Capryltriglycerid, Cetylricinoleat, Cholesterolhydroxystearat, Cholesterolisostearat,

$C_1$-$C_{30}$-Monoester und Polyester von Glycerin, beispielsweise Glyceryltribehenat, Glycerylstearat, Glycerylpalmitat, Glyceryldistearat, Glyceryldipalmitat, $C_1$-$C_{30}$-Carbonsäuremonoester und Polyester von Zucker, beispielsweise Glucosetetraoleat, Glucosetetraester der Sojaölfettsäure, Mannosetetraester der Sojaölfettsäure, Galactosetetraester der Ölsäure, Arabinosetetraester der Linolsäure, Xylosetetralinoleat, Galactosepentaoleat, Sorbitoltetraoleat, Sorbitolhexaester der ungesättigten Sojaölfettsäure, Xylitolpentaoleat, Sucrosetetraoleat, Sucrosepentaoleat, Sucrosehexaoleat, Sucroseheptaoleat, Sucroseoleat.

An Silikonölen stehen vorzugsweise zur Verfügung Dimethylpolysiloxane und Cyclomethicone, Polydialkylsiloxane $R_3SiO(R_2SiO)_xSiR_3$, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, Trimethylsiloxysilicate $[(CH_2)_3SiO)_{1/2}]_x[SiO_2]_y$, wobei x für eine Zahl von 1 bis 500 und y für eine Zahl von 1 bis 500 steht, Dimethiconole $R_3SiO[R_2SiO]_xSiR_2OH$ und $HOR_2Sio1R_2SiO]_xSiR_2OH$, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die Haarfärbe- und -tönungsmittel enthalten vorzugsweise Direktfarbstoffe und/oder Oxidationsfarbstoffvorstufen in den üblichen pH-Bereichen.

An Direktfarbstoffen in Betracht kommen vorzugsweise Nitroanilinderivate, wie 1-[(2-Hydroxyethyl)-amino]-2-nitrobenzol (Velsol™ Yellow 2), 4-Hydroxypropylamino-3-Nitrophenol (Velsol™ Red BN), 3-Nitro-p-Hydroxyethylaminophenol (Velsol™ Red 54), 4-Hydroxyethylamino-3-Nitroanilin (Velsol™ Red 3), N,N'-Bis-(Hydroxyethyl)-2-Nitro-p-Phenylendiamine (Velsol™ Violet BS), N,N',N'-Tris-(Hydroxyethyl)-2-Nitro-p-Phenylendiamine (Velsol™ Blue 2), 4-(2'-Hydroxyethyl)-amino-3-nitro-toluol, 4-(2'-Hydroxyethyl)-amino-3-nitro-benzylalkohol, 4-(2'-Hydroxyethyl)-amino-3-nitro-1-trifluormethylbenzol, 4-(2',3'-Dihydroxypropylramino-3-nitro-chlorbenzol, 4-(2'-Hydroxyethyl)-amino-3-nitro-brombenzol und 4-(2',3'-Dihydroxypropyl)-amino-3-nitro-brombenzol, Nitrobenzolderivate, beispielsweise 2-Amino-4-nitro-phenol, Pikraminsäure, 1-[(2'-Hydroxyethyl)-amino]-2-amino-4-nitrobenzol, 2-Nitro-4-[(2'-hydroxyethyl)amino]-anilin, 4-Bis-[(2'-hydroxyethyl)amino]-1-methylamino-2-nitrobenzol, 2,5-Bis-[(2'-hydroxyethyl)-amino]-nitrobenzol, 2-(2'-hydroxyethyl)-amino-4,6-dinitro-phenol, 1-Amino-4-(2',3'-dihydroxypropyl)-amino-2-nitro-5-chlor-benzol, aber auch Triphenylmethanfarbstoffe, wie zum Beispiel Basic Violet 1 (C.I. 42535), Azofarbstoffe, wie zum Beispiel Acid Brown 4 (C.I. 14805), Anthrachinonfarbstoffe, wie zum Beispiel Disperse Blue 23 (C.I. 61545), Disperse Violet 4 (C.1.61105), 1,4,5,8-Tetraaminoanthrachinon und 1,4-Diaminoanthrachinon und weitere direktziehende Farbstoffe.

An Oxidationsfarbstoffvorstufen stehen vorzugsweise zur Verfügung p-Phenylendiamine und p-Aminophenole oder deren Derivate wie beispielsweise p-Toluylendiamin, p-Phenylendiamin, p-Aminophenol, die zum Zweck der Nuancierung der Färbung mit sogenannten Modifiern oder Kupplern, wie zum Beispiel m-Phenylendiamin, Resorcin, m-Aminophenol und deren Derivaten kombiniert werden.

Als Oxidationsmittel zur Entwicklung der Haarfärbungen kommen vorzugsweise Hydrogenperoxyd und dessen Additionsverbindungen in Betracht.

Zur Erhöhung der Farbintensität können die Mittel die in kosmetischen Systemen üblichen Carrier enthalten, insbesondere Benzylalkohol, Vanillin (4-Hydroxy-3-methoxy-benzaldehyd), Isovanillin, p-Hydroxyanisol, 3-Hydroxy-4-methoxybenzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoesäuremethylester, p-Hydroxybenzaldehyd, m-Kresol, Hydrochinonmonomethylether, o-Fluorphenol, m-Fluorphenol, p-Fluorphenol, 2-(2'-Hydroxyphenoxy)-ethanol, 3,4-Methylendioxyphenol, Resorcinmonomethylether, 3,4-Dimethoxyphenol, 3-Trifluormethylphenol. Resorcinmonoacetat, Ethylvanillin, 2-Thiophenethanol, Milchsäurebutylester und Glycolsäurebutylester. Besonders vorteilhaft mit synergistischer Wirkung sind Mittel, enthaltend Phenoxyethanol oder/oder Benzylalkohol.

Die Haarfärbemittel können vorteilhafterweise perlglanzgebende Verbindungen enthalten, beispielsweise Fettsäuremonoalkanolamide, Fettsäuredialkanolamide, Monoester oder Diester von Alkylenglycol, insbesondere Ethylenglykol und/oder Propylenglykol oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art, bevorzugt Ethylenglycoldistearat und Polyethylenglycoldistearat mit ca. 3 Glykoleinheiten.

Die Haarbehandlungsmittel enthalten bevorzugt 0,1 bis 15, besonders bevorzugt 1 bis 10 Gew.-% an perlglanzgebenden Verbindungen.

Glitter- und Glanzeffekte der Mittel lassen sich vorzugsweise durch Zugabe von Glimmer, colorierte Polyacrylester und Glimmer, Glimmer-Eisenoxid, Glimmer-Titanoxid und durch Pigmente erzeugen. Als Pigmente eignen sich Metalloxide, beispielsweise Eisenoxide, Titanoxid, Ultramarinblau, sowie mit kationischen Coatinghüllen modifizierte Pigmente, wie in WO 00/12053 und EP 504 066 beschrieben.

Die kosmetischen Mittel können als weitere Hilfs- und Zusatzstoffe Tenside, Emulgatoren, kationische Polymere, Verdicker, Filmbildner, antimikrobielle Wirkstoffe, Adstringentien, Antioxidation, UV-Lichtschutzfilter, Pigmente/Mikropigmente, Gelierungsmittel, sowie weitere in der Kosmetik gebräuchliche Zusätze, wie z.B. Überfettungsmittel, feuchtigkeitsspendende Mittel, Silicone, Stabilisatoren, Konditioniermittel, Glycerin, Konservierungsmittel, Perlglanzmittel, Farbstoffe, Duft - und Parfümöle, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistik, Antiallergica, Corticosteroide, Sebostatika), Vitamine, Bisabolol®, Allantoin®, Phytantriol®, Panthenol®, AHA-Säuren, Pflanzenextrakte, beispielsweise Aloe Vera und Proteine enthalten.

Als anionische waschaktive Substanzen seien vorzugsweise genannt: $C_{10}$-$C_{20}$-Alkyl-und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfate, Alkansulfonate und Hydroxyalkansulfonate, Olefinsulfonate, Acylester von Isethionaten, $\alpha$-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate- oder -glycinate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammoniumsowie analogen Alkylammonium-Salze.

Der Gewichtsanteil der anionischen Tenside beträgt bevorzugt 1 bis 30 Gew.-%, besonders bevorzugt 5 bis 25 Gew.-%, insbesondere bevorzugt 10 bis 22 Gew.-%, bezogen auf die fertigen Mittel.

Geeignete kationische Tenside sind beispielsweise quartäre Ammonium-Salze wie Di-($C_{10}$-$C_{24}$-Alkyl)-dimethylammonium-chlorid oder -bromid, vorzugsweise Di-($C_{12}$-$C_{18}$-Alkyl)-dimethyl-ammonium-chlorid oder -bromid; $C_{10}$-$C_{24}$-Alkyl-dimethylethylammonium-chlorid oder -bromid; $C_{10}$-$C_{24}$-Alkyl-trimethyl-ammonium-chlorid oder -bromid, vorzugsweise Cetyl-trimethyl-ammonium-chlorid oder -bromid und $C_{20}$-$C_{22}$-Alkyl-trimethyl-ammonium-chlorid oder -bromid; $C_{10}$-$C_{24}$-Alkyl-dimethylberizylammonium-chlorid oder -bromid, vorzugsweise $C_{12}$-$C_{18}$-Alkyl-dimethylbenzylammonium-chlorid; N-($C_{10}$-$C_{18}$-Alkyl)-pyridinium-chlorid oder -bromid, vorzugsweise N-($C_{12}$-$C_{16}$-Alkyl)-pyridinium-chlorid oder -bromid; N-($C_{10}$-$C_{18}$-Alkyl)-isochinoliniumchlorid, -bromid oder -monoalkylsulfat; N-($C_{12}$-$C_{18}$-Alkylpolyoylaminoformylmethyl)-pyridinium-chlorid; N-($C_{12}$-$C_{18}$-Alkyl)-N-methyl-morpholinium-chlorid, -bromid oder -monoalkylsulfat; N-($C_{12}$-$C_{18}$-Alkyl)-N-ethyl-morpholinium-chlorid, -bromid oder- monoalkylsulfat; $C_{16}$-$C_{18}$-Alkyl-pentaoxethyl-ammonium-chlorid; Diisobutylphenoxyethoxyethyldimethylbenzylammonium-chlorid; Salze des N,N-Diethylamino-ethylstearylamids und -oleylamids mit Salzsäure, Essigsäure, Milchsäure, Zitronensäure, Phosphorsäure; N-Acyl-aminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside beträgt bevorzugt 0,1 bis 10 Gew.-%, besonders bevorzugt 0,2 bis 7 Gew.-%, insbesondere besonders bevorzugt 0,5 bis 5 Gew.-% bezogen auf das fertige Mittel.

Als nichtionische Tenside, die als waschaktive Substanzen eingesetzt werden können, kommen vorzugsweise in Betracht Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics®); Fettsäureamidpolyethylenglykole; N-Alkyl-, N-Alkoxypolyhydroxyfettsäureamid, insbesondere Fettsäure-N-methylglucamide, Saccharoseester; Polyglykolether, Alkylpolyglycoside, Phosphorsäureester (Mono-, Di- und Triphosphorsäureester ethoxyliert und nichtethoxyliert).

Der Gewichtsanteil der nichtionischen Tenside in den Mitteln (z.B. im Falle von Rinse-off-Produkten) liegt bevorzugt im Bereich von 1 bis 20 Gew.-%, besonders bevorzugt 2 bis 10 Gew.-%, insbesondere bevorzugt 3 bis 7 Gew.-% bezogen auf das fertige Mittel.

Bevorzugte Amphotenside sind: N-($C_{12}$-$C_{18}$-Alkyl)-$\beta$-aminopropionate und N-($C_{12}$-$C_{18}$-Alkyl)-$\beta$-iminodipropionate als Alkali- und Mono-, Di- und Trialkylammonium-Salze; N-Acylaminoalkyl-N,N-dimethyl-acetobetain, vorzugsweise N-($C_8$-$C_{18}$-Acyl)aminopropyl-N,N-dimethylacetobetain; $C_{12}$-$C_{18}$-Alkyl-dimethyl-sulfopropyl-betain; Amphotenside auf Basis Imidazolin (Handelsname: Miranol®, Steinapon®), vorzugsweise das Natrium-Salz des 1-($\beta$-Carboxy-methyloxyethyl)-1-(carboxymethyl)-2-lauryl-imidazoliniums; Aminoxide, z.B. $C_{12}$-$C_{18}$-Alkyldimethylaminoxid, Fettsäureamidoalkyldimethylaminoxid.

Der Gewichtsanteil der amphoteren Tenside beträgt bevorzugt 0,5 bis 20 Gew.-%, besonders bevorzugt 1 bis 10 Gew.-% bezogen auf das fertige Mittel.

Des weiteren können in den Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den Mitteln sind Alkylethersulfate, Alkylsulfate, insbesondere Laurylsulfat, Alkylbetaine, insbesondere Cocoamidopropylbetain, Amphoacetate, Acylglutamate, insbesondere Natriumcocoylglutamat, Alkylethersulfosuccinate, insbesondere Di-natriumlaureth-sulfosuccinat und Cocosfettsäurediethanolamid.

Die Gesamtmenge der in den Mitteln eingesetzten Tenside beträgt vorzugsweise 1 bis 70 Gew.-%, besonders bevorzugt 10 und 40 Gew.-%, insbesondere bevorzugt 12 bis 35 Gew.-%, bezogen auf das fertige Mittel.

Als Emulsionen vorliegende Mittel können ohne weiteren Emulgator erzeugt werden oder auch einen oder mehrere Emulgatoren enthalten. Diese Emulgatoren können gewählt werden aus der Gruppe der nichtionischen, anionischen, kationischen oder amphoteren Emulgatoren.

Als nichtionogene Co-Emulgatoren kommen vorzugsweise in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; ($C_{12}$-$C_{18}$)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z.B. anionische Emulgatoren, wie mono-, di- oder tri-Phosphorsäureester, Seifen (z.B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Weiterhin können natürlich vorkommende Emulgatoren, unter denen Bienenwachs, Wollwachs, Lecithin und Sterole bevorzugt sind, verwendet werden.

Vorzugsweise sind Fettalkoholethoxylate gewählt aus der Gruppe der ethoxylierten Stearylalkohole, Cetylalkohole, Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol (18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether; Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol (1 4)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether, Polyethylenglycol(20)cetylstearylether, Polyethylenglycol(20)stearat, Polyethylenglycol(21 )stearat, Polyethylenglycol(22)stearat, Polyethylenglycol(23)stearat, Polyethylenglycol(24)stearat, Polyethylenglycol(25)stearat, Polyefhylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol (25)isostearat, Polyethylenglycol(12)oleat, Polyethylenglycol(13)oleat, Polyethylenglycol(14)oleat, Polyethylenglycol (15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure oder deren Salze kann vorteilhafterweise das Natriumlaureth-11-carboxylat verwendet werden.

Als Alkylethersulfat ist Natrium Laureth-1-4-sulfat, als ethoxyliertes Cholesterinderivat Polyethylenglycol(30) Cholesterylether vorteilhaft. Ebenso bevorzugt ist Polyethylenglycol(25)Sojasterol.

Als ethoxylierte Triglyceride können vorteilhaft Polyethylenglykol(60)Evening Primose Glyceride verwendet werden.

Weiterhin ist es von Vorteil, die Polyethylenglycolglycerinfettsäureester aus der Gruppe Polyethylenglycol(20) glyceryllaurat, Polyethylenglycol(6)glycerylcaprat/caprinat, Polyethylenglycol(20)glyceryloleat, Polyethylenglycol(20) glycerylisostearat und Polyethylenglycol(1 8)glyceryloleat/cocoat zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat, Polyethylenglycol (20)sorbitanmonooleat.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atomen, Diglycerinester gesättigter und/oder ungesättigter, verzweigter und/oder unverzweigter Alkancarbonsäuren einer Kettenlänge von 8 bis 24, insbesondere 12 bis18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen, sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.
Insbesondere vorteilhafte W/O-Emulgatoren sind Glycerylmonostearat, Glycerylmonoisostearat, Glycerylmonomyristat, Glycerylmonooleat, Glycerylmonolaurat, Glycerylmonocaprylat, Glycerylmonocaprinat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol oder Polyethylenglycol(2)stearylether.

Der Gewichtsanteil des oder der in den Mitteln enthaltenen Emulgators oder Emulgatoren, zusätzlich zum Aminoorganopolysiloxan ($S_H$), beträgt vorzugsweise 0,1 bis 20 Gew.-%, besonders bevorzugt 0,5 bis 15 Gew.-%, insbesondere bevorzugt 1 bis 10 Gew.-% bezogen auf das fertige Mittel.

Als kationische Polymere eignen sich vorzugsweise die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (®Salcare SC95), PVP-dimethylaminoethylmethacrylat-Copolymer, Guarhydroxypropyltriammoniumchloride, sowie Calciumalginat und Ammoniumalginat.

Des weiteren können vorzugsweise eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxypropyldiethylentriamin; Polyaminopolyamid und kationische Chitinderivate, wie beispielsweise Chitosan.

Der Gewichtsanteil an kationischen Polymeren in den Mitteln kann vorzugsweise im Bereich von 0,1 bis 10 Gew.-%, besonders bevorzugt im Bereich von 0,2 bis 5 Gew.-%, insbesondere bevorzugt im Bereich von 0,5 bis 2,5 Gew.-% liegen.

Die gewünschte Viskosität der Mittel kann durch Zugabe von Verdickungsmitteln eingestellt werden. In Betracht kommen vorzugsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester.

Als synthetische Polymere kommen verschiedene Materialien zum Einsatz, vorzugsweise Polyvinylalkohole, Polyacrylamide, Polyvinylamide, Polysulfonsäuren, insbesondere Copolymerisate auf Basis von Ammoniumsalzen von Acrylamidoalkylsulfonsäuren und cyclischen N-Vinylcarbonsäureamiden bzw. cyclischen und linearen N-Vinylcarbonsäureamiden oder auch hydrophob modifizierte Acrylamidoalkylsulfonsäure-Copolymerisate, Polyacrylsäure, Polyacrylsäurederivate, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den obengenannten Verbindungen, einschließlich ihrer verschiedenen Salze und Ester. Diese Polymere können wahlweise vernetzt oder unvernetzt sein.

Insbesondere für Mittel auf Ölbasis besonders geeignete Verdicker sind Dextrinester, beispielsweise Dextrinpalmitat, aber auch Fettsäureseifen, Fettalkohole und Silikonwachse, beispielsweise Alkylmethicone, SilCare® 41.M40; SilCare® 41 M50, SilCare® 41M65, SilCare® 41M70 oder SilCare® 41M80.

Bevorzugte Filmbildner sind je nach Anwendungszweck Salze der Phenylbenzimidazolsulfonsäure, wasserlösliche Polyurethane, beispielsweise $C_{10}$-Polycarbamylpolyglycerylester, Polyvinylalkohol, Polyvinylpyrrolidoncopolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan, Vinylacetat/Crotonat, beispielsweise unter dem Handelsnamen Aristoflex® A 60 (Clariant) erhältlich, sowie polymere Aminoxide, beispielsweise unter den Handelsnamen Diaformer Z-711, 712, 731, 751 erhältliche Vertreter.

Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die Mittel enthalten die antimikrobiellen Mittel bevorzugt in Mengen bis 50 Gew.-%, besonders bevorzugt in Mengen von 0,01 bis 10 Gew.-%, insbesondere bevorzugt in Mengen von 0,1 bis 10 Gew.-%.

Bevorzugte Adstringentien sind Oxide, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink.

Die Mittel enthalten die adstringenden Wirkstoffe bevorzugt in Mengen von 0 bis 50 Gew.-%, besonders bevorzugt in Mengen von 0,01 bis 10 Gew.% und insbesondere bevorzugt in Mengen von 0,1 bis 10 Gew.-%.
Vorteilhafte Mittel enthalten ein oder mehrere Antioxidantien. Als günstige, aber dennoch fakultativ zu verwendende Antioxidantien können alle für kosmetische und/oder pharmazeutische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. (α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, *y*-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den Mitteln beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Mittel.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Mittel, zu wählen.

In einer besonders bevorzugten Ausführungsform der Erfindung enthalten die kosmetischen oder pharmazeutischen Mittel Antioxidantien ausgewählt aus Superoxid-Dismutase, Tocopherol (Vitamin E) und Ascorbinsäure (Vitamin C).

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäure-isoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Als Pigmente/Mikropigmente können vorzugsweise mikrofeines Titandioxid, Glimmer-Titanoxid, Eisenoxide, Glimmer-Eisenoxid, Zinkoxid, Siliziumoxide, Ultramarinblau, Chromoxide, eingesetzt werden.

Als Gelierungsmittel eignen sich alle oberflächenaktiven Stoffe, die in der flüssigen Phase gelöst eine Netzwerkstruktur ausbilden und so die flüssige Phase verfestigen. Geeignete Geliermittel sind z.B. in WO 98/58625 genannt.

Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher.

Bevorzugt enthalten die Mittel 0,01 bis 20 Gew.%, besonders bevorzugt 0,1 bis 10 Gew.-%, insbesondere bevorzugt 1 bis 8 Gew.-% und ganz besonders bevorzugt 3 bis 7 Gew.-% an Geliermitteln.
Weitere Zusatzstoffe können Siliconverbindungen sein, vorzugsweise Dimethylpolysiloxan, Methyl-phenylpolysiloxane, cyclische Silicone sowie amino-, fettsäure-, alkohol-, polyether-, epoyx-, fluor- und/oder alkylmodifizierte Siliconverbindungen, beispielsweise Alkylsilicone SilCare® Silicone 41M10, SilCare® Silicone 41 M15, SilCare® Silicone 41 M20, SilCare® Silicone 41 M30 (Clariant), Alkyltrimethicone SilCare® 31 M30, SilCare® 31 M40, SilCare® 31 M 50, SilCare® 31 M 60 (Clariant), Phenyltrimethicone SilCare® 15M30, SilCare® 15M40, SilCare® 15M50, SilCare® 15M60 (Clariant), Polyalkylarylsiloxane und Polyethersiloxan-Copolymere.

Die Mittel können die oben genannten Siliconverbindungen vorzugsweise in den Gewichtsmengen von 0,1 bis 20 Gew.-%, besonders bevorzugt 0,2 bis 15 Gew.-%, insbesondere bevorzugt 0,5 bis 10 Gew.-% bezogen auf die fertigen Mittel enthalten.

Als Trägermaterialien in Betracht kommen vorzugsweise pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als fungizide Wirkstoffe können vorzugsweise Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Octopirox in den Gewichtsmengen von 0,05 bis 5 Gew.-%, bevorzugt 0,1 bis 3 Gew.-%, besonders bevorzugt 0,2 bis 2 Gew.-%, bezogen auf die fertigen Mittel eingesetzt werden.

Die Mittel können vorteilhaft mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden und ähnlichen Stoffen, abgemischt werden.

Als perlglanzgebende Verbindungen bevorzugt sind Fettsäuremonoalkanolamide, Fettsäuredialkanolamide, Monoester oder Diester von Alkylenglycol, insbesondere des Ethylenglykols und/oder Propylenglycols oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art. In den Mitteln sind als pedglanzgebende Komponente besonders bevorzugt Ethylenglycoldistearat und Polyethylenglycoldistearat mit 3 Glykoleinheiten.

Als feuchtigkeitsspendende Substanz stehen vorzugsweise Isopropylpalmitat, Glycerin und/oder Sorbitol zu Verfügung, die bevorzugt in den Gewichtsmengen 0,1 bis 50 % eingesetzt werden.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als Konservierungsmittel in Betracht kommen vorzugsweise Phenoxyethanol, Parabene, Pentandiol oder Sorbinsäure. Sie werden vorzugsweise in den Gewichtsmengen von 0,001 bis 5 Gew.-%, besonders bevorzugt von 0,01 bis 3 Gew.-%, insbesondere bevorzugt von 0,1 bis 2 Gew.-%, bezogen auf die fertigen Mittel eingesetzt.

Als Farbstoffe können die für kosmetische und pharmazeutische Zwecke geeigneten und zugelassenen Substanzen verwendet werden.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, -p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18C-Atomen, Citral, Citronellal, Citronellyloxyacetaldehyd, Cycllamenaldehyd, Hydroxycitronellal, Lilial und Bourgeonal, zu den Ketonen z.B. die lonone, alpha-Isomethylionon und Methyl-cedrylketon, zu den Alkoholen Anethol, Citronellol, Eugenol, Geranion, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Säuren oder Laugen zur pH-Wert Einstellung werden vorzugsweise Mineralsäuren, beispielsweise HCl, anorganische Basen, beispielsweise NaOH, KOH und organische Säuren, bevorzugt Zitronensäure verwendet.

Die Mittel sind vorzugsweise auf einen pH Wert im Bereich 2 bis 12, bevorzugt pH 3 bis 8, eingestellt.

Die kosmetischen und pharmazeutischen Mittel können unter Verwendung der substituierten Aminopolyorganosiloxane ($S_H$) hergestellt werden.

Zur Herstellung der kosmetischen oder pharmazeutischen Mittel kann vorzugsweise ein Konzentrat enthaltend 70 bis 99,99 Gew.%, besonders bevorzugt 70 bis 99 Gew.-% und insbesondere bevorzugt 75 bis 95 Gew.-% einer oder mehrerer substituierter Aminoorganopolysiloxane ($S_H$), bezogen auf das fertige Konzentrat, verwendet werden.

Die nachfolgenden Beispiele und Anwendungen sollen die Erfindung näher erläutern, ohne sie jedoch darauf zu beschränken (bei allen Prozentangaben handelt es sich um Gewichtsprozent).

Beispiel 1: Herstellung von SilCare® Silicone SEA

951,76 Teile Octamethylcyclotetrasiloxan sowie 38,31 Teile [N-(2-Aminoäthyl)-3-aminopropyl]-methyl-dimethoxysilan werden mit 0,95 Teilen Tetrabutylammoniumhydroxyd (40 %-ige methanolische Lösung) versetzt und unter leichtem Stickstoffstrom innerhalb 90 Minuten auf 70°C aufgeheizt. Nach 2 Stunden bei 70°C wird bis auf einen Restdruck von 50 mbar evakuiert und anschließend bei konstantem Restdruck auf 110°C aufgeheizt. Nach einer Stunde bei 110°C und 50 mbar, unter Vakuum wird auf Raumtemperatur abgekühlt. Überschüssiges Octamethylcyclotetrasiloxan wird abdestilliert und es werden ca. 965,00 Teile aminomodifiziertes Polydimethylsiloxan (S) mit einer Aminzahl von ca. 0,385 erhalten.

193,00 Teile (S) werden unter Rühren und Stickstoff mit 87,00 Teilen Alkylpolyglykolglycidyläther (H) gemischt, auf 150°C aufgeheizt und ca. 8 Stunden bei 150°C bis zur vollständigen Umsetzung von (H) gerührt. Das Reaktionsprodukt wird abgekühlt und isoliert. Man erhält 280,00 Teile Silikonöl ($S_H$). 280,00 Teile Silikonöl ($S_H$) werden bei Raumtemperatur mit 28,00 Teilen Tridecanolpoly-9,5-glykoläther und 14 Teilen Wasser vermischt. Man erhält 322,00 Teile-SilCare®-Silicone-SEA in Form einer transparenten, viskosen, leicht mit Wasser weiterverdünnbaren Flüssigkeit mit einem pH-Wert von ca. 9,0.

Beispiel 2: Anwendungsbeispiel Farbschutz

Standardisierte, blond gebleichte Haarsträhnen wurden mit einer handelsüblichen Permanenthaarfarbe (Viva Purered, Glutrot) unter Standardbedingungen gefärbt. Anschließend wurde die Strähne A mit Ethersulfat:Betain (3:1, 12 % AI) und die Strähne B mit Ethersulfat:Betain (3:1, 12 % AI) + SilCare® Silicone SEA (1 % AI) je 4x gewaschen. In dem 10 Personen umfassenden Panel wird die Strähne A als Standard (0) gesetzt. Eine Verbesserung gegenüber dem Standard wird mit + oder ++ (sehr gut) bewertet, eine Verschlechterung mit - oder - -.

Nachfolgend sind die Parameter Farbintensität, Farbbrillance, Glanz, Griff und elektrostatische Aufladung nach den vier Waschvorgängen aufgeführt (Durchschnitt aus allen Testpersonen).

| | Farbintensität | Farbbrillance | Glanz | Griff | elektrostatische Aufladung |
|---|---|---|---|---|---|
| Strähne A (Standard) | 0 | 0 | 0 | 0 | 0 |
| Strähne B | + | ++ | ++ | + | 0 |

Ergebnis:

Die mit SilCare® Silicone SEA behandelten Haarsträhnen weisen nach optischer und sensorischer Prüfung ein deutliches geringeres Ausbluten der Haarfarbe auf (erhöhte Farbintensität, höhere Farbbrillance) und zeigen zusätzlich einen signifikant verbesserten Glanz und einen besseren Griff.

Beispiel 3: W/O-Creme

| | | | |
|---|---|---|---|
| A | Hostacerin® DGI | Clariant | 4,00 % |
| | Bienenwachs | | 2,00 % |
| | Lunacera® M | | 3,00 % |
| | Magnesiumstearat | | 1,00 % |
| | Mineralöl, niedrigviskos | | 5,00 % |
| | Vaseline | | 10,00 % |
| | Cetiol® V | | 5,00 |
| | SilCare® Silicone SEA | | 1,00 % |
| B | 1,2-Propylenglycol | | 3,00 % |
| | Wasser | | ad 100 % |
| | Konservierungsmittel | | q.s. |
| C | Duftstoff | | 0,40 % |

Herstellweise:

I Schmelzen von A bei 80°C
II Erwärmen von B auf 80°C
III Einrühren von II in I
IV Rühren bis Temperatur von 35°C erreicht ist
V Zugabe von C zu IV bei 35°C

Beispiel 4: O/W-Creme

| | | | |
|---|---|---|---|
| A | Hostacerin® DGI | Clariant | 2,00 % |
| | Isopropylpalmitat | | 4,00 % |
| | Octyldodecanol | | 4,00 % |
| | NIPAGUARD® PDU | Clariant | q.s. |
| | SilCare® Silicone SEA | Clariant | 1,00 % |
| B | ARISTOFLEX® AVC | Clariant | 1,20 % |
| C | Hostapon® KCG | Clariant | 0,80 % |
| | Wasser | | ad 100 % |
| D | Duftstoff | | 0,40 % |

Herstellweise:

I Einrühren von B in A
II Einrühren von D in I
III Homogenisieren

Beispiel 7: Creme-Spülung

| | | | |
|---|---|---|---|
| A | Hostacerin® DGI | Clariant | 1,50 % |
| | Cetylalkohol | | 3,00 % |
| B | Genamin® CTAC | Clariant | 3,30 % |
| | Wasser | | ad 100 % |

(fortgesetzt)

| | | | |
|---|---|---|---|
| | Konservierungsmittel | | q.s. |
| C | Duftstoff | | 0,30 % |
| | SilCare® Silicone SEA | Clariant | 1,00 % |

Herstellweise:

I Schmelzen von A bei ca. 75°C
II Erwärmen von B auf ca. 75°C
III Zugabe von II zu I unter Rühren und weiterrühren bis 30°C
IV Zugabe von C zu III bei 30°C
V Einstellen auf pH 4,0 mit Zitronensäure

Beispiel 8: Haarshampoo

| | | | |
|---|---|---|---|
| A | Genapol® LRO flüssig | Clariant | 31,10 % |
| | Duftstoff | | 0,30 % |
| B | Wasser | | ad 100 % |
| | SilCare® Silicone SEA | Clariant | 1,00 % |
| | Genagen® CAB | Clariant | 12,00 % |
| | Duftstoff | | q.s. |
| | Konservierungsmittel | | q.s. |
| C | NaCl | | 6,00 % |

Herstellweise:

I Mischen der Komponenten A
II Nacheinander Zugabe der Komponenten B zu I
III Einstellen des pH-Wertes
IV Einstellen der Viskosität mit C

Beispiel 9: Shampoo mit Farbschutz für gefärbtes Haar

| | | | |
|---|---|---|---|
| A | Glucamat DOE-120 | | 2,00 % |
| | Emulsogen® HCO 040 | Clariant | 2,00 % |
| B | Wasser | | ad 100 % |
| C | Genapol® LRO flüssig | Clariant | 22,22 % |
| | Genagen® KB | Clariant | 13,33 % |
| | Genamin® KSL | Clariant | 3,33 % |
| | Aristoflex® PEA 70 | Clariant | 2,86 % |
| | Sandopan® DTC, Säure | Clariant | 2,20 % |
| | NIGAGUARD® DCB | Clariant | 0,10 % |
| | SilCare® Silicone SEA | Clariant | 0,50 % |
| | Farbstoff | | q.s. |
| | Duftstoff | | 0,20 % |
| D | NaOH | | |

Herstellweise:

I Einrühren der Komponenten A in B und Erwärmen auf ca. 60°C und unter Rühren Abkühlen auf Raumtemperatur
II Nacheinander Einrühren der Komponenten C in I
III Rühren bis Formulierung klar erscheint
IV Einstellen auf pH 5,5 mit D

Beispiel 10: Tönungsshampoo

| | | | |
|---|---|---|---|
| A | Genagen® KB | Clariant | 7,00 % |
| | Velsol Semipermanentfarbstoff | Clariant | 0,50 % |
| B | Genapol® T 500 P | Clariant | 0,50 % |
| | Wasser | | ad 100 % |
| C | Genapol® LRO flüssig | Clariant | 30,00 % |
| | Genagen® LAA | Clariant | 3,00 % |
| | Genamin® CTAC | Clariant | 1,00 % |
| | SilCare® Silicone SEA | Clariant | 0,50 % |
| | Tetranatrium EDTA | | 0,10 % |
| | NIGAGUARD® DMDMH | Clariant | 0,30 % |
| | Genapol® PDB | Clariant | 3,00 % |
| | Kaliumphosphat | | 1,50 % |
| D | Zitronensäure | | |

Herstellweise:

I Lösen der Komponenten unter Rühren
II Mischen der Komponenten B und Erwärmen bis zur klaren Lösung
III Abkühlen von B auf ca. 35°C und nacheinander Zugabe der Komponenten C zu II
IV Einrühren von I in III
V Einstellen auf pH 5,5 mit D

Beispiel 11: Haar-Gel

| | | | |
|---|---|---|---|
| A | Aristoflex® AVC | Clariant | 1,40 % |
| | Wasser | | ad 100 % |
| B | Diaformer Z-751 | | 3,00 % |
| | Alkohol denat. | | 30,00 % |
| | Genapol® C100 | Clariant | 0,40 % |
| | Duftstoff | | 0,20 % |
| C | Farbstoff | | q.s. |
| | Phenonip® | Clariant | 0,50 % |
| D | SilCare® Silicone SEA | Clariant | 0,50 % |

Herstellweise:

I Lösen der Komponenten A
II Mischen der Komponenten B
III Zugabe von II zu I unter Rühren
IV Zugabe von C zu III
V Zugabe von D zu IV

Beispiel 12: Haarspitzenpflege

| | | | |
|---|---|---|---|
| A | Wasser | | 50,0 % |
| B | Tylose® H 100000 G4 | | 1,00 % |
| C | Wasser | | ad 100 % |
| D | Genamin® PDAC | Clariant | 2,50 % |
| | Glycerin | | 2,00 % |
| | SilCare® Silicone SEA | Clariant | 1,00 % |
| E | Zitronensäure | | q.s. |

Herstellweise:

I B in A aufquellen
II Nacheinander Auflösen der einzelnen Komponenten von D in C
III Zugabe von II zu I
IV Einstellen des pH-Wertes mit E

Chem. Bezeichnung der eingesetzten Handelsprodukte

| | | |
|---|---|---|
| Aristoflex® AVC | (Clariant) | Ammoniumacryloyldimethyl-Taurat/NVP Copolymer (NVP: N-Vinylpyrrolidon) |
| Aristoflex® HMB | (Clariant) | Ammoniumacryloyldimethyl-Taurat/Beheneth-25 Methacrylat Polymer |
| Aristoflex® PEA 70 | (Clariant) | Polypropylenterephthalat |
| Cetiol® V | (Cognis) | Decyloleat |
| Diaformer Z-751 | | Lauryl-/Stearylacrylat, Ethylenaminoxid, Methacrylat Copolymer |
| Emulsogen® HCO 040 | (Clariant) | PEG-40 Hydrogenated Castor Oil |
| Extrapon Avocado special | | Wasser/ Ethoxydiglycol/ Propylenglycol/ Butylenglycol/Persea Gratissima Extrakt |
| Genagen® CAB | (Clariant) | Cocoamidopropylbetain |
| Genagen® KB | (Clariant) | Cocobetain |
| Genagen® LAA | (Clariant) | Natriumlauroamphoacetat |
| Genamin® CTAC | (Clariant) | Cetrimoniumchlorid |
| Genamin® KSL | (Clariant) | PEG-5 Stearylammoniumlactat |
| Genamin® PDAC | (Clariant) | Polyquaternium-6 |
| Genapol® C100 | (Clariant) | Coceth-10 |
| Genapol® PDB | (Clariant) | Glycoldistearat/Laureth-4/ Cocoamidopropylbetain |
| Genapol® LRO fl. | (Clariant) | Natriumlaurethsulfat |
| Genapol® T 500 P | (Clariant) | Ceteareth-50 |
| Glucamat DOE-120 | | PEG-120 Methylglusosedioleat |
| Hostacerin® DGI | (Clariant) | Polyglyceryl-2-Sesquiisostearat |
| Hostapon® KCG | (Clariant) | Natriumcocoylglutamat |
| Locron® L | (Clariant) | Aluminium Chlorohydrat |
| Lunacera® M | (H.B. Fuller) | mikrokristallines Wachs |

(fortgesetzt)

| | | |
|---|---|---|
| NIPAGUARD® CMB | (Clariant) | Triethylenglycol/Benzylalkohol/ Propylenglycol/Chloromethyliso-thiazolinon/Methylisothiazolinon |
| NIPAGUARD DCB | (Clariant) | Phenoxyethanol, Methyl-dibromglutaronitril |
| NIGAGUARD® DMDMH | (Clariant) | DMDM Hydantoin |
| NIPAGUARD® PDU | (Clariant) | Propylen glycol/Diazolidinylharnstoff/ Methylparaben/Propylparaben |
| Octopirox® | (Clariant) | Pirocton Olamin |
| Phenonip® | (Clariant) | Phenoxyethanol/ Methyl-/Ethyl-/ Butyl-/Propyl-/Isobutylparaben |
| Sandopan® DTC, Säure | (Clariant) | Trideceth-7-Carbonsäure |
| SilCare® 1 M75 | (Clariant) | Retinoxytrimethylsilan |
| SilCare® 15M50 | (Clariant) | Phenyltrimethicon |
| SilCare® 31 M50 | (Clariant) | Caprylyltrimethicon |
| SilCare® 41 M15 | (Clariant) | Caprylylmethicon |
| SilCare® Silicone SEA | (Clariant) | Beispiel 1 |
| Softigen® 767 | (Sasol) | PEG-6 Caprylic/Capric Glycerid |
| Tylose® H 100000 G4 | | Hydroxyethylcellulose |

## Patentansprüche

**1.** Verwendung eines kosmetischen oder pharmazeutischen Mittels enthaltend ein oder mehrere substituierte Aminopolyorganosiloxane ($S_H$) mit substituierten Aminogruppen, die über Alkylenbrücken oder Mono- oder Oligo-(alkylenamino)-alkylenbrücken an Siliciumatome des Polysiloxangrundgerüstes gebunden sind, wobei
die in den Aminopolyorganosiloxanen ($S_H$) vorkommenden Aminogruppen mindestens teilweise mit einem Rest der Formel ($\varepsilon$)

$$T\text{-}CH_2\text{-}CHOH\text{-}CH_2\text{-} \qquad (\varepsilon),$$

worin T den Rest eines tensiden Monoalkoholpolyglykoläthers mit Emulgatorcharakter bedeutet und die Alkoholpolyglykoläther T-H solche der folgenden durchschnittlichen Formel

$$R_1 \leftarrow\!\!\!( O - X )_q\, OH \qquad \textbf{(II)}$$

sind,
worin

$R_1$ einen alkylaromatischen oder aliphatischen Kohlenwasserstoffrest mit 9 bis 24 Kohlenstoffatomen,
X $C_{2-4}$-Alkylen

und

q 4 bis 50,

bedeuten, wobei mindestens 80 % der q Alkylenoxygruppen Äthylenoxyeinheiten sind, substituiert sind,
die in den Aminopolyorganosiloxanen ($S_H$) vorkommenden Aminogruppen im durchschnittlichen Verhältnis von mindestens 1,5 Resten der Formel ($\varepsilon$) pro Sigebundene Aminoalkylgruppe oder Amino-mono- oder -oligo-(alkylenamino)-alkylGruppe substituiert sind, und
vorhandene Aminogruppen gegebenenfalls mindestens teilweise zu Amidgruppen acyliert und/oder alkyliert und/oder benzyliert und/oder protoniert sind,

zum Schutz und zur Erhaltung der Farbe in gefärbten Keratinfasern.

2. Verwendung eines kosmetischen oder pharmazeutischen Mittels gemäß Anspruch 1, **dadurch gekennzeichnet, dass** das kosmetische oder pharmazeutische Mittel ein oder mehrere Substanzen ausgewählt aus Konservierungsmitteln und Duft - und Parfümölen enthält.

3. Verwendung eines kosmetischen oder pharmazeutischen Mittels gemäß Anspruch 1 oder 2, **dadurch gekennzeichnet, dass** das substituierte Aminopolyorganosiloxan ($S_H$) einen Stickstoffgehalt im Bereich von 0,03 bis 4,2 Gew.% aufweist.

4. Verwendung eines kosmetischen oder pharmazeutischen Mittels gemäß einem oder mehreren der Ansprüche 1 bis 3, **dadurch gekennzeichnet, dass** die gesamten Aminogruppen des substituierten Aminopolyorganosiloxans ($S_H$) zu 40 bis 100 % durch Reste der Formel (ε) substituiert sind.

5. Verwendung eines kosmetischen oder pharmazeutischen Mittels gemäß einem oder mehreren der Ansprüche 1 bis 4, **dadurch gekennzeichnet, dass** das substituierte Aminopolyorganosiloxan ($S_H$) durch Umsetzung von Aminopolyorganosiloxan (S), welches primäre und/oder sekundäre Aminogruppen, die über Alkylenbrücken oder Mono- oder Oligo-(alkylenamino)-alkylenbrücken an Siliciumatome des Polysiloxangrundgerüstes gebunden sind, enthält, mit mindestens einem Alkoholpolyglykoläther-monoglycidyläther (H) und gegebenenfalls anschließender Acylierung und/oder Alkylierung und/oder Benzylierung und/oder Protonierung, erhalten wurde.

6. Verwendung eines kosmetischen oder pharmazeutischen Mittels gemäß Anspruch 5, **dadurch gekennzeichnet, dass** das Aminopolyorganosiloxan (S) eine Aminzahl im Bereich von 0,05 bis 3 aufweist.

7. Verwendung eines kosmetischen oder pharmazeutischen Mittels gemäß einem oder mehreren der Ansprüche 1 bis 6, **dadurch gekennzeichnet, dass** das substituierte Aminopolyorganosiloxan ($S_H$) ein durchschnittliches Molekulargewicht im Bereich von 15000 bis 2000000 aufweist.

8. Verwendung eines kosmetischen oder pharmazeutischen Mittels gemäß einem oder mehreren der Ansprüche 1 bis 7, **dadurch gekennzeichnet, dass** $R^1$ 11 bis 24 Kohlenstoffatome enthält.

9. Verwendung eines kosmetischen oder pharmazeutischen Mittels gemäß einem oder mehreren der Ansprüche 1 bis 7, **dadurch gekennzeichnet, dass** $R^1$ 9 bis 22 Kohlenstoffatome enthält.

10. Verwendung eines kosmetischen oder pharmazeutischen Mittels gemäß einem oder mehreren der Ansprüche 1 bis 9, **dadurch gekennzeichnet, dass** $R^1$ 11 bis 22 Kohlenstoffatome enthält.

11. Verwendung eines kosmetischen oder pharmazeutischen Mittels gemäß einem oder mehreren der Ansprüche 1 bis 10, **dadurch gekennzeichnet, dass** q im Bereich von 4 bis 30 ist.

12. Verwendung eines kosmetischen oder pharmazeutischen Mittels gemäß einem oder mehreren der Ansprüche 1 bis 11, **dadurch gekennzeichnet, dass** 100 % der q Alkylenoxyeinheiten Äthylenoxyeinheiten sind.

13. Verwendung eines kosmetischen oder pharmazeutischen Mittels nach einem oder mehreren der Ansprüche 1 bis 12 zum Schutz und zur Erhaltung der Farbe in gefärbtem menschlichem Haar.

14. Verwendung nach einem oder mehreren der Ansprüche 1 bis 13, **dadurch gekennzeichnet, dass** das Mittel von 0,01 bis 10 Gew.-% bezogen auf das fertige Mittel an substituiertem Aminopolyorganosiloxan ($S_H$) enthält.

## Claims

1. The use of a cosmetic or pharmaceutical composition comprising one or more substituted aminopolyorganosiloxanes ($S_H$) with substituted amino groups which are bonded to silicon atoms of the polysiloxane basic structure via alkylene bridges or mono- or oligo(alkylenamino)alkylene bridges, where the amino groups present in the aminopolyorganosiloxanes ($S_H$) are substituted at least partially by a radical of the formula (ε)

$$T—CH_2—CHOH—CH_2— \qquad (\varepsilon),$$

in which T is the radical of a surfactant monoalcohol polyglycol ether with emulsifier character and the alcohol polyglycol ethers T-H are those of the following average formula

$$R_1\text{-}(O\text{-}X)_q\text{-}OH \qquad (II)$$

in which $R_1$ is an alkylaromatic or aliphatic hydrocarbon radical having 9 to 24 carbon atoms,
X is $C_{2-4}$-alkylene
and q is 4 to 50,
where at least 80% of the q alkylenoxy groups are ethylenoxy units,
the amino groups present in the aminopolyorganosiloxanes ($S_H$) are substituted in the average ratio of at least 1.5 radicals of the formula
(ε) per Si-bonded aminoalkyl group or amino-mono- or - oligo-(alkylenamino)-alkyl group, and
present amino groups are, if appropriate, acylated and/or alkylated and/or benzylated and/or protonated at least partially to give amide groups,
for protecting and preserving the color in colored keratin fibers.

2. The use of a cosmetic or pharmaceutical composition as claimed in claim 1, wherein the cosmetic or pharmaceutical composition comprises one or more substances selected from preservatives and fragrance and perfume oils.

3. The use of a cosmetic or pharmaceutical composition as claimed in claim 1 or 2, wherein the substituted aminopolyorganosiloxane ($S_H$) has a nitrogen content in the range from 0.03 to 4.2% by weight.

4. The use of a cosmetic or pharmaceutical composition as claimed in one or more of claims 1 to 3, wherein all of the amino groups of the substituted aminopolyorganosiloxane ($S_H$) are 40 to 100% substituted by radicals of the formula (ε).

5. The use of a cosmetic or pharmaceutical composition as claimed in one or more of claims 1 to 4, wherein the substituted aminopolyorganosiloxane ($S_H$) has been obtained by reaction of aminopolyorganosiloxane (S) which comprises primary and/or secondary amino groups which are bonded to silicon atoms of the polysiloxane basic structure via alkylene bridges or mono- or oligo(alkylenamino)alkylene bridges, with at least one alcohol polyglycol ether monoglycidyl ether (H) and, if necessary, subsequent acylation and/or alkylation and/or benzylation and/or protonation.

6. The use of a cosmetic or pharmaceutical composition as claimed in claim 5, wherein the aminopolyorganosiloxane (S) has an amine number in the range from 0.05 to 3.

7. The use of a cosmetic or pharmaceutical composition as claimed in one or more of claims 1 to 6, wherein the substituted aminopolyorganosiloxane ($S_H$) has an average molecular weight in the range from 15 000 to 2 000 000.

8. The use of a cosmetic or pharmaceutical composition as claimed in one or more of claims 1 to 7, wherein $R^1$ comprises 11 to 24 carbon atoms.

9. The use of a cosmetic or pharmaceutical composition as claimed in one or more of claims 1 to 7 wherein $R^1$ comprises 9 to 22 carbon atoms.

10. The use of a cosmetic or pharmaceutical composition as claimed in one or more of claims 1 to 9 wherein $R^1$ comprises 11 to 22 carbon atoms.

11. The use of a cosmetic or pharmaceutical composition as claimed in one or more of claims 1 to 10 wherein q is in the range from 4 to 30.

12. The use of a cosmetic or pharmaceutical composition as claimed in one or more of claims 1 to 11, wherein 100% of the q alkylenoxy units are ethylenoxy units.

13. The use of a cosmetic or pharmaceutical composition as claimed in one or more of claims 1 to 12 for protecting and preserving the color in colored human hair.

14. The use as claimed in one or more of claims 1 to 13, wherein the composition comprises from 0.01 to 10% by weight, based on the finished composition, of substituted aminopolyorganosiloxane ($S_H$).

## Revendications

1. Utilisation d'une composition cosmétique ou pharmaceutique contenant un ou plusieurs aminopolyorganosiloxanes substitués ($S_H$) à groupes amino substitués qui sont liés par des ponts alkylène ou des ponts mono- ou oligo-(alkylèneamino)-alkylène à des atomes de silicium du squelette de base polysiloxane,
les groupes amino apparaissant dans les aminopolyorganosiloxanes ($S_H$) étant au moins en partie substitués par un radical de formule (ε)

$$T\text{-}CH_2\text{-}CHOH\text{-}CH_2\text{-} \qquad (\varepsilon),$$

dans laquelle T représente le reste d'un monoalcool-polyglycoléther tensioactif à caractère d'émulsifiant et les alcoolpolyglycoléthers T-H sont ceux de formule moyenne suivante

$$R_1\text{-}(\text{-}O\text{-}X\text{-})_q\text{-}OH \qquad (II),$$

dans laquelle

$R_1$ représente un radical hydrocarboné aliphatique ou alkylaromatique ayant de 9 à 24 atomes de carbone,
X représente un groupe alkylène en $C_2$-$C_4$

et

q vaut de 4 à 50,

au moins 80 % des groupes oxyalkylène q étant des unités oxyde d'éthylène,
les groupes amino apparaissant dans les aminopolyorganosiloxanes ($S_H$) étant substitués dans le rapport moyen d'au moins 1,5 radical de formule ($_E$) par groupe aminoalkyle ou groupe amino-mono- ou -oligo-(alkylèneamino)-alkyle, lié à Si, et
les groupes amino présents étant éventuellement au moins en partie acylés en groupes amido et/ou alkylés et/ou benzylés et/ou protonés,
pour la protection et pour le maintien de la couleur dans des fibres de kératine colorées.

2. Utilisation d'une composition cosmétique ou pharmaceutique selon la revendication 1, **caractérisée en ce que** la composition cosmétique ou pharmaceutique contient une ou plusieurs substances choisies parmi les conservateurs et huiles essentielles et huiles parfumées.

3. Utilisation d'une composition cosmétique ou pharmaceutique selon la revendication 1 ou 2, **caractérisée en ce que** l'aminopolyorganosiloxane substitué ($S_H$) présente une teneur en azote dans la plage de 0,03 à 4,2 % en poids.

4. Utilisation d'une composition cosmétique ou pharmaceutique selon une ou plusieurs des revendications 1 à 3, **caractérisée en ce que** les groupes amino en totalité de l'aminopolyorganosiloxane substitué ($S_H$) sont substitués à raison de 40 à 100 % par des radicaux de formule (ε).

5. Utilisation d'une composition cosmétique ou pharmaceutique selon une ou plusieurs des revendications 1 à 4, **caractérisée en ce que** l'aminopolyorganosiloxane substitué ($S_H$) a été obtenu par mise en réaction d'aminopolyorganosiloxane (S) qui contient des groupes amino primaires et/ou secondaires qui sont liés par des ponts alkylène ou des ponts mono- ou oligo-(alkylèneamino)-alkylène à des atomes de silicium du squelette de base polysiloxane, avec au moins un alcoolpolyglycoléthermonoglycidyléther (H) et éventuellement acylation et/ou alkylation et/ou benzylation et/ou protonation subséquente(s).

6. Utilisation d'une composition cosmétique ou pharmaceutique selon la revendication 5, **caractérisée en ce que** l'aminopolyorganosiloxane (S) présente un indice d'amine dans la plage de 0,05 à 3.

7. Utilisation d'une composition cosmétique ou pharmaceutique selon une ou plusieurs des revendications 1 à 6,

**caractérisée en ce que** l'aminopolyorganosiloxane substitué ($S_H$) présente une masse moléculaire moyenne dans la plage de 15 000 à 2 000 000.

**8.** Utilisation d'une composition cosmétique ou pharmaceutique selon une ou plusieurs des revendications 1 à 7, **caractérisée en ce que** $R^1$ contient de 11 à 24 atomes de carbone.

**9.** Utilisation d'une composition cosmétique ou pharmaceutique selon une ou plusieurs des revendications 1 à 7, **caractérisée en ce que** $R^1$ contient de 9 à 22 atomes de carbone.

**10.** Utilisation d'une composition cosmétique ou pharmaceutique selon une ou plusieurs des revendications 1 à 9, **caractérisée en ce que** $R^1$ contient de 11 à 22 atomes de carbone.

**11.** Utilisation d'une composition cosmétique ou pharmaceutique selon une ou plusieurs des revendications 1 à 10, **caractérisée en ce que** q est dans la plage de 4 à 30.

**12.** Utilisation d'une composition cosmétique ou pharmaceutique selon une ou plusieurs des revendications 1 à 11, **caractérisée en ce que** 100 % des unités oxyde d'alkylène q sont des unités oxyde d'éthylène.

**13.** Utilisation d'une composition cosmétique ou pharmaceutique selon une ou plusieurs des revendications 1 à 12, pour la protection et le maintien de la couleur dans le cheveu humain teint.

**14.** Utilisation selon une ou plusieurs des revendications 1 à 13, **caractérisée en ce que** la composition contient de 0,01 à 10 % en poids, par rapport à la composition finale, d'aminopolyorganosiloxane substitué ($S_H$).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5075403 A
- WO 02092666 A
- WO 0012053 A
- EP 504066 A
- WO 9858625 A